# EUROPEAN PATENT SPECIFICATION

(11) **EP 4 405 496 B1**
(45) Date of publication and mention of the grant of the patent: **04.11.2026**
(21) Application number: 21958559.3
(22) Date of filing: 23.09.2021
(51) Int. Cl.: C12Q 1/6806, C12Q 1/6869, C12Q 1/6858

(54) **COMPOSITIONS AND METHODS FOR TARGETED NGS SEQUENCING OF CFRNA AND CFTNA**
ZUSAMMENSETZUNGEN UND VERFAHREN ZUR GEZIELTEN NGS-SEQUENZIERUNG VON CFRNA UND CFTNA
COMPOSITIONS ET PROCÉDÉS POUR LE SÉQUENÇAGE CIBLÉ PAR NGS DE CFARN ET CFANT

(43) Date of publication of application: 31.07.2024
(73) Proprietor: Genomic Testing Cooperative, LCA, Irvine, CA 92618 (US)
(72) Inventor: ALBITAR, Maher, Valley Center, California 92082 (US)
(74) Representative: Hutter, Anton
(86) International application number: PCT/US2021/051683
(87) International publication number: WO 2023/048713

(56) References cited:
- WO-A1-2019/113563
- WO-A1-2020/092646
- WO-A1-2020/132144
- WO-A2-2018/126278
- US-A1- 2003 186 287
- US-A1- 2010 035 243
- US-A1- 2016 024 556
- US-A1- 2018 327 831
- US-A1- 2019 185 930
- KRUG A.K. ET AL: "Improved EGFR mutation detection using combined exosomal RNA and circulating tumor DNA in NSCLC patient plasma", vol. 29, no. 3, 1 March 2018 (2018-03-01), pages 700 - 706, XP093227195, ISSN: 0923-7534, Retrieved from the Internet <URL:https://pmc.ncbi.nlm.nih.gov/articles/PMC5889041/pdf/mdx765.pdf> DOI: 10.1093/annonc/mdx765
- MOKÁNSZKI ATTILA, BICSKÓ RÉKA, GERGELY LAJOS, MÉHES GÁBOR: "Cell-Free Total Nucleic Acid-Based Genotyping of Aggressive Lymphoma: Comprehensive Analysis of Gene Fusions and Nucleotide Variants by Next-Generation Sequencing", CANCERS, vol. 13, no. 12, pages 3032, XP093052467, DOI: 10.3390/cancers13123032

## Description

### Field of the Invention

The field of the invention is compositions and methods for analysis of cell-free nucleic acids from various biological fluids, and especially as it relates to cell-free RNA (cfRNA) and cell-free DNA (cfDNA) from plasma and serum.

### Background of the Invention

The background description includes information that may be useful in understanding the present invention. It is not an admission that any of the information provided herein is prior art or relevant to the presently claimed invention, or that any publication specifically or implicitly referenced is prior art.

Cell-free nucleic acids (cfNA), and especially cell-free DNA (cfDNA) and cell-free RNA (cfRNA) present in blood and other biological fluids were more recently proposed as potential markers to detect diseased cells and tissue in a subject, such as cancer cells or tumors. To that end, circulating nucleic acids need to be isolated form the biological fluid, and various kits and methods are known in the art to achieve such isolation. For example, cfDNA and/or cfRNA can be isolated using solid phase (typically silica-based) adsorption and subsequent clean-up to remove non-nucleic acid components (e.g., QIAamp Circulating Nucleic Acid Kit or Apostle MiniMax High Efficiency cfDNA_RNA (cfNAs) Isolation Kit) or using an aqueous two-phase system as described in WO 2021/037075. Alternatively, circulating cfDNA or cfRNA can also be isolated using a microfluidic device (see *e.g.,* NPJ Precision Oncology (2020)4:3). In yet further examples, US 2014/0356877 teaches nucleic acid isolation from blood using electrochemical separation, and US 2015/0031035 teaches circularization of nucleic acids and subsequent rolling circle amplification. Regardless of the manner of preparation, the so obtained nucleic acid preparation is then subjected to further analysis.

For example, US 2006/0228727 teaches analyzing together the quantity of DNA and RNA of certain genes in plasma/serum of cancer patients as an overall reflection of gene amplification and/or gene over expression in comparison to healthy controls. While conceptually relatively simple, such method will not provide mutation-specific information and also identify whether or not a mutation in a DNA segment of a cell is transcribed. In another example of sequence analysis (see *e.g.,* US 2020/0199671), cfRNA and cellular RNA are sequenced, and the cellular RNA sequence information is used to filter cfRNA sequence information. Such approach can advantageously exclude cellular RNA contamination in cfRNA samples, analysis is limited to RNA information only. WO 2018/208892 teaches RNA expression profiling using circulating tumor RNA, once more limiting analysis to RNA. Similarly, US 2020/0232010 teaches a method of cfDNA analysis that is based on size distribution and fragmentation to so reduce sample bias. However, such method only analyzes cfDNA in a sample.

In an effort to analyze both DNA and RNA, US 2019/0390253 describes analysis of multiple forms (here: dsDNA, ssDNA, ssRNA) and/or modifications of nucleic acid in a sample using a form-specific sequence tag, such that sequence information can be obtained for distinct forms encoding the same gene. In addition, such method also allows for form-specific amplification and enrichment. While such analysis advantageously allows for concurrent analysis of DNA and RNA, sensitivity of such assays is expected to be relatively low, especially where the DNA and/or RNA is present at low copy numbers/transcripts. Moreover, sensitivity is even more problematic where the DNA and/or RNA are isolated from plasma or serum. In at least some instances, sequencing libraries from cell free nucleic acids can be improved by use of small capture probes as is described in US 2018/032783 1. However, such approach is typically limited to the population of nucleic acids already isolated and as such will not increase sensitivity, especially where the gene or transcript of interest is subject to low copy numbers or translation and has high instability as is often the case with mutant genes and mutant transcripts.

Thus, even though various systems and methods of isolation and analysis of circulating nucleic acids are known in the art, all or almost all of them suffer from several drawbacks. Therefore, there remains a need for compositions and methods for isolation and analysis of circulating nucleic acids, especially where the circulating nucleic acids are isolated form blood and have low stability. Krug, A.K. et al., ("Improved EGFR mutation detection using combined exosomal RNA and circulating tumor DNA in NSCLC patient plasma", 2018, Ann Oncol 29:700-706) discloses that a major limitation of circulating tumor DNA (ctDNA) for somatic mutation detection has been the low level of ctDNA found in a subset of cancer patients, and investigates whether using a combined isolation of exosomal RNA (exoRNA) and cell-free DNA (cfDNA) could improve blood-based liquid biopsy for EGFR mutation detection in non-small-cell lung cancer (NSCLC) patients. US 2016/024556 A1 discloses a method of obtaining an enriched population of nucleic acids of interest from a test sample, comprising providing a mixture of nucleic acids comprising genomic DNA sequences and cDNA sequences obtained from the test sample, contacting the mixture of nucleic acids with a set of probes under a condition sufficient for hybridization of said nucleic acids to said probes, wherein said probes are complementary to nucleic acids of interest present in said mixture of nucleic acids, and separating nucleic acids hybridized to said probes from those not hybridized, thereby obtaining an enriched population of nucleic acids of interest. US 2019/185930 A1 discloses a method for preparing a sequencing library of a sample comprising cell-free nucleic acids wherein the methods involve DNase treating an extracted nucleic acid sample to remove cell-free DNA from a mixed cfDNA and cfRNA test sample. WO 2018/126278 A2 discloses the differential treatment of a sample to obtain fractions either comprising DNA or RNA for use in producing a sequencing library. WO 2019/113563 A1 discloses a method of preparing a cDNA library comprising providing a sample containing RNA, isolating polynucleotides from the sample, degrading initial DNA in the isolated polynucleotides with a DNase to produce an RNA-enriched sample.

### Summary of The Invention

The invention is as defined by the claims, and any other aspects, configurations, or embodiments set forth herein not falling within the scope of the claims are for information only. Additionally, any references in the description to methods of treatment refer to the compounds, pharmaceutical compositions, and medicaments of the present invention for use in a method of treatment of the human (or animal) body by therapy (of for diagnosis).

The inventive subject matter is directed to various compositions and methods of improved isolation and analysis of circulating cell free nucleic acids in biological fluids, and especially in blood of a subject.

Especially preferred compositions and methods employ both a cfTNA and a cfRNA fraction from the same sample fluid, wherein the fractions are obtained in a process that allows for isolation of degraded nucleic acids (e.g., having fragment sizes of 100 or less nucleotides). Moreover, after reverse transcription of both fractions, preferred methods further enrich the so prepared cDNA libraries in a target-specific manner using multiple hybridization probes for amplification for each target cDNA such that the hybridization probes bind to the same target cDNA in a tiled fashion.

Notably, sequence analysis of thusly prepared target-enriched cDNA libraries from the cfTNA and cfRNA fractions provided unprecedented sensitivity and specificity with respect to multiple genes of interest. Indeed, the inventor demonstrated that not only presence of various cancers can be detected in a blood sample, but that such methods also allow for cancer classification (e.g., type or stage of cancer).

In one aspect of the inventive subject matter, the inventor contemplates a method of manipulating nucleic acids from a cell-free fluid that includes a step of obtaining cell-free total nucleic acid (cfTNA) from a biological fluid, and a further step of subjecting a first portion of the cfTNA to DNAse digestion to so generate a cfRNA fraction of the cfTNA. In yet another step, both the cfRNA fraction of the cfTNA and a second portion of the cfTNA are subjected to reverse transcription, adapter ligation, and amplification to thereby generate respective first and second cDNA libraries, and each of the first and second cDNA libraries are then subjected to target enrichment that enriches a plurality of target cDNAs to thereby generate respective first and second target-enriched cDNA libraries.

In some embodiments, the cfTNA comprises cfRNA fragments having a size of between 17 and 200 bases, and cfDNA fragments having a size of between 50 and 300 bases, and/or the cfTNA comprises cfRNA fragments having a size of between 30 and 250 bases, and cfDNA fragments having a size of between 75 and 400 bases. In further contemplated embodiments, the cfRNA fragments and the cfDNA fragments may constitute together at least 30% or at least 40% of all cfTNA.

While not limiting to the inventive subject matter, the step of obtaining the cfTNA from the biological fluid may be performed by simultaneous isolation of cfRNA and cfDNA. Additionally, or alternatively, it is contemplated that the step of reverse transcription will include a step of random priming for the first strand synthesis, and/or a step of incorporating dUTP into the second strand synthesis. Most typically, but not necessarily, adapter ligation may include a step of ligating adapters having a 3'-dTMP overhang. It is further preferred (especially where NGS sequencing is employed) that the adapter ligation will use adapters that comprise a p5 sequence portion, a p7 sequence portion, a first index sequence portion, a second index sequence portion, a first sequencing primer binding site sequence portion, and/or a second sequencing primer binding site sequence portion. Most typically, the amplification will be performed over between 6-15 amplification cycles.

In still further embodiments, the target enrichment will use for each target cDNA a plurality of hybridization probes that bind to the target cDNA at respective different positions. Therefore, in some aspects the plurality of hybridization probes will bind to the target cDNA in a tiled fashion (e.g., with a tiling density of at least 2x). Viewed from a different perspective, the plurality of hybridization probes may bind to the target cDNA in a tiled fashion with a step length of n, wherein n is an integer between 1-10. Regardless of the specific tiling, it is generally preferred that each of the plurality of hybridization probes has a length of 100-150 bases. As will be readily appreciated, first and the second target-enriched cDNA libraries may be further amplified for sequencing, record keeping, etc.

Therefore, contemplated methods will also include a step of sequencing the first and the second target-enriched cDNA libraries or the amplified first and the second target-enriched cDNA libraries to thereby generate first and second sequence data sets, respectively. As will also be readily recognized, the first and second datasets will typically include sequence information as well as provide quantitative information (e.g., TPM data or copy number data).

In another aspect of the inventive subject matter, which falls outside the subject matter of the claims, the inventor contemplates a method of detecting mutations in cfTNA with increased sensitivity that includes a step of obtaining from a sample of a biological fluid cfRNA and cfTNA, and a further step of generating from the cfRNA and cfTNA respective first and second cDNA libraries. In still another step, each of the first and second cDNA libraries each are subjected to target enrichment that enriches a plurality of target cDNAs to thereby generate respective first and second target-enriched cDNA libraries, and in yet another step, the first and second target-enriched cDNA libraries are sequenced (e.g., using NGS sequencing). The sequencing results from the first and second target-enriched cDNA libraries are then used to thereby detect mutations with increased sensitivity as compared to sequencing cfRNA or cfDNA from the same sample alone.

Most typically, but not necessarily, the step of obtaining the cfTNA from the biological fluid uses simultaneous isolation of cfRNA and cfDNA. In such and other methods, it is generally preferred that the cfTNA comprises cfRNA fragments having a size of between 17 and 200 bases, and cfDNA fragments having a size of between 50 and 300 bases, or that the cfTNA comprises cfRNA fragments having a size of between 30 and 250 bases, and cfDNA fragments having a size of between 75 and 400 bases. Viewed from a different perspective, it is contemplated that the cfRNA fragments and the cfDNA fragments constitute together at least 30% or at least 40% of all cfTNA.

It is still further contemplated that the target enrichment uses for each target cDNA a plurality of hybridization probes that bind to the target cDNA at respective different positions. For example, the plurality of hybridization probes may bind to the target cDNA in a tiled fashion, preferably with a tiling density of at least 2x. Therefore, the plurality of hybridization probes may bind to the target cDNA in a tiled fashion with a step length of n, wherein n is an integer between 1-10. Among other options, it is generally preferred that each of the plurality of hybridization probes has a length of 100-150 bases.

Additionally, it is contemplated that the step of sequencing comprises paired-end sequencing, and/or that the sequencing is performed to a read depth of at least 20x. In contemplated methods, the step of detecting mutations detects at least one of a single nucleotide change, an insertion of one or more nucleotides, a deletion of one or more nucleotides, an inversion, a translocation, and copy number variation. Moreover, contemplated methods also allow for determination of a variant allele fraction. Advantageously, detection of unique mutations and/or sensitivity of variant allele fraction detection is increased as compared to cfDNA alone.

In a further aspect of the inventive subject matter, which falls outside the subject matter of the claims, the inventor also contemplates reagent kit for sequence analysis that may include a first reagent comprising a cfDNA-depleted cfRNA fraction of cfTNA of a biological fluid and a second reagent comprising cfTNA of the same biological fluid. Most typically, the biological fluid is human plasma or serum. For example, the first reagent may comprise cfRNA fragments predominantly having a size of between 17 and 200 bases and cfDNA fragments predominantly having a size of between 50 and 300 bases, and/or the second reagent comprises cfRNA fragments predominantly having a size of between 17 and 200 bases. Most typically, the cfRNA fragments and the cfDNA fragments constitute together at least 30% or at least 40% of all cfTNA. In some embodiments, the first reagent may be prepared from the second reagent.

In yet another aspect of the inventive subject matter, which falls outside the subject matter of the claims, the inventor contemplates a reagent kit for sequence analysis that may include a first target-enriched cDNA library and a second target-enriched cDNA library, wherein the first target-enriched cDNA library does not comprise a cfDNA fraction of cfTNA of a biological fluid, and wherein the second target-enriched cDNA library comprises a cfDNA fraction of cfTNA of the same biological fluid.

Where desired, the first and second target enriched cDNA libraries are target enriched using the same target cDNAs, and/or the target cDNA encodes a cancer associated gene, a cell signaling associated gene, an immunophenotype associated gene, or a receptor associated gene. It is still further contemplated that respective cDNAs of the first and second target enriched cDNA libraries may comprise at least one of a p5 sequence portion, a p7 sequence portion, a first index sequence portion, a second index sequence portion, a first sequencing primer binding site sequence portion, and a second sequencing primer binding site sequence portion. Advantageously, the cDNAs of the first and/or second target enriched cDNA libraries represent at least 90% of all nucleic acids present in the biological fluid that correspond to the target cDNA.

Therefore, in still another aspect of the inventive subject matter, which falls outside the subject matter of the claims, the inventor contemplates a reagent kit for sequence analysis that includes a plurality of nanoparticles having a surface and size that allows binding of RNA having a size of equal or less than 50 bases and that allows binding of DNA having a size of equal or less than 100 bases. Such kits will further include a plurality of target enrichment oligonucleotides having sequence complementarity to a target gene, wherein at least some of the target enrichment oligonucleotides hybridize to distinct portions of the same target gene.

In at least some embodiments, the plurality of nanoparticles may have a surface and size that allows binding of RNA having a size of equal or less than 30 bases and that allows binding of DNA having a size of equal or less than 80 bases, or may have a surface and size that allows binding of RNA having a size of equal or less than 20 bases and that allows binding of DNA having a size of equal or less than 60 bases. Most typically, but not necessarily, the plurality of nanoparticles are paramagnetic nanoparticles. With respect to the target enrichment oligonucleotides it is typically preferred that the plurality of target enrichment oligonucleotides comprise for each target cDNA a plurality of hybridization probes that bind to the target cDNA at respective different positions. For example, the plurality of hybridization probes may bind to the target cDNA in a tiled fashion, wherein the plurality of hybridization probes provide a tiling density of at least 2x. Thus, suitable hybridization probes may bind to the target cDNA in a tiled fashion with a step length of n, wherein n is an integer between 1-10. In further examples, each of the plurality of hybridization probes may have a length of 100-150 bases. Additionally, contemplated kits may also include at least one of a reverse transcriptase, a ligase, and a plurality of distinct adapters suitable for paired-end sequencing.

Consequently, the inventor also contemplates in still another aspect of the inventive subject matter, which falls outside the subject matter of the claims, a method of analyzing nucleic acid data of a subject that includes a step of sequencing a first target-enriched cDNA library and a second target-enriched cDNA library to thereby obtain respective first and second sequence data sets. Most typically, the first target-enriched cDNA library is prepared from cfTNA and does not comprise a cfDNA fraction of cfTNA of a biological fluid of the subject, and the second target-enriched cDNA library is prepared from cfTNA and does comprise a cfDNA fraction of cfTNA of the same biological fluid. In a further step of such method, one or mutations are identified for each gene in the first and second sequence data sets, and expression levels are determined for at least one gene in at least the first sequence data set. In some embodiments, the step of sequencing is paired-end sequencing.

It should be noted that use of first and second target-enriched cDNA libraries increase sensitivity of detection of mutations as compared to detection of mutations of the first target-enriched cDNA library alone. Preferably, but not necessarily, the first and second target-enriched cDNA libraries are enriched for a target cDNA that encodes a cancer associated gene, a cell signaling associated gene, an immunophenotype associated gene, or a receptor associated gene, and optionally the first and second target-enriched cDNA libraries are enriched for a target cDNA that is specific for specific disease for diagnosis or determination of a clinical course, response to a therapy, or relapse of the disease.

Moreover, it is contemplated that such methods may also include a step of using the first and second sequence data sets in a machine learning algorithm to identify one or more genes associated with a disease parameter. For example, suitable disease parameters are presence of a cancer, type of cancer, recurrence of cancer, and/or or residual cancer. Additionally, or alternatively, it is contemplated that such methods may include a step of using the first and second sequence data sets in a machine learning algorithm to identify one or more genes associated with a cytogenetic parameter (e.g., translocation and/or loss or duplication of at least a portion of a chromosome). Likewise, it is contemplated that such methods may include a step of using the first and second sequence data sets in a machine learning algorithm to identify one or more genes associated with an immunohistochemical parameter (e.g., presence or quantity of a cell surface receptor and/or presence or quantity of a cell surface enzyme), and/or that such methods may include a step of using the first and second sequence data sets in a model to thereby identify a disease parameter, a cytogenetic parameter, and/or an immunohistochemical parameter. As will be readily appreciated, such methods may further include a step of administering a treatment based on the one or more mutations and/or quantified expression.

Consequently, outside the subject matter of the claims, the inventors also contemplate a method of classifying a cancer in a subject that includes a step of sequencing (e.g., using paired-end sequencing sequencing) a first target-enriched cDNA library and a second target-enriched cDNA library to thereby obtain respective first and second sequence data sets. Preferably, the first target-enriched cDNA library does not comprise a cfDNA fraction of cfTNA of a biological fluid of the subject, whereas the second target-enriched cDNA library comprises a cfDNA fraction of cfTNA of the same biological fluid. In a further step of such method, one or more mutations are identified for each gene in the first and second sequence data sets, and an expression level is quantified for one or more genes in at least the first sequence data set. The so identified mutation and quantified expression level can then be used in a trained model to thereby classify the cancer in the subject.

In some embodiments, the first and second target-enriched cDNA libraries are enriched for a target cDNA that encodes a cancer associated gene, a cell signaling associated gene, an immunophenotype associated gene, or a receptor associated gene. For example, the trained model may classify the cancer as being present, being recurrent, or being residual, or the trained model may classify the cancer as a solid cancer, a sarcoma, or a lymphoma. Most typically, the trained model is constructed using machine leaning with a Bayesian classifier. As should be readily apparent, contemplated methods may also include a step of administering a treatment based on the classification of the cancer.

Therefore, and viewed from a different perspective, the inventor contemplates, outside the subject matter of the claims, a method of treating a subject that includes a step of sequencing (e.g., using paired-end sequencing) a first target-enriched cDNA library and a second target-enriched cDNA library to thereby obtain respective first and second sequence data sets. Preferably, the first target-enriched cDNA library does not comprise a cfDNA fraction of cfTNA of a biological fluid of the subject, whereas the second target-enriched cDNA library comprises a cfDNA fraction of cfTNA of the same biological fluid. A further step of such methods includes identifying, for each gene in the first and second sequence data sets one or more mutations, and quantifying for each gene an expression level in at least the first sequence data set. A treatment is then administered based on the identified mutation and quantified expression level.

As before, it is contemplated that the first and second target-enriched cDNA libraries are enriched for a target cDNA that encodes a cancer associated gene, a cell signaling associated gene, an immunophenotype associated gene, or a receptor associated gene. Therefore, the treatment may comprise administering a chemotherapeutic agent, an immune stimulatory agent, a checkpoint inhibitor, and/or a cancer vaccine. It should also be appreciated that the treatment will preferably be based on a model (e.g., Bayesian classifier-trained model) that uses the identified mutation and quantified expression level.

Lastly, outside the subject matter of the claims, the inventor contemplates a reagent kit for sequence analysis of cDNA obtained from a biological fluid that includes a plurality of target enrichment probes that hybridize to respective target cDNAs, wherein the target cDNAs encode cancer associated genes, cell signaling associated genes, immunophenotype associated genes, and/or receptor associated genes. Where desired, each of the target enrichment probes may further comprise a sequence portion for solid phase capture, a chemical modification for solid phase capture, or a magnetic bead. Most typically, the target cDNAs are prepared from cfTNA and cfRNA of the biological fluid. In some embodiments, the target cDNA encodes a gene of Table 1 below.

Various objects, features, aspects and advantages of the inventive subject matter will become more apparent from the following detailed description of preferred embodiments, along with the accompanying drawing figures in which like numerals represent like components.

### Brief Description of The Drawing

**FIG.1** is an exemplary graph depicting mutation count using cfRNA, cfTNA, and cfDNA in samples using target enrichment as described herein.
**FIG.2** is an exemplary graph depicting variant allele frequency (VAF) using cfTNA and cfDNA in samples using target enrichment as described herein.
FIG.3 is an exemplary graph depicting variant allele frequency (VAF) using cfRNA and cfTNA in samples using target enrichment as described herein.
FIG.4 is an exemplary graph detecting variant allele frequency (VAF) detection using cfRNA as compared with cfTNA.
FIG.5 is an exemplary graph depicting relative expression of CCND1 to CD22 as a diagnostic tool for mantle cell lymphoma.
FIG.6 is an exemplary graph depicting relative expression of CCND1 to CD22 as a diagnostic tool for chronic lymphocytic lymphoma.
FIG.7 is an exemplary graph depicting expression of MUC1 as a diagnostic tool for a solid cancer (breast cancer).
FIG.8 is an exemplary graph depicting expression of HER2 as a diagnostic tool for a solid cancer (breast cancer).
FIG.9 is an exemplary graph of a trained model for general cancer detection (all types) using target enrichment as described herein.
FIG.10 is an exemplary graph of a trained model for specific cancer subtype detection (lymphoid neoplasms) using target enrichment as described herein.
FIG.11 is an exemplary graph of a trained model for specific cancer subtype detection (myeloid neoplasms) detection using target enrichment as described herein.
FIG.12 is an exemplary graph of a trained model for specific cancer subtype detection (solid neoplasms) detection using target enrichment as described herein.
**FIG.13** is an exemplary graph of a trained model for specific cancer subtype detection (solid neoplasms) detection using target enrichment and TPM/CNV data as described herein.
**FIG.14** is an exemplary graph of a trained model for specific cancer subtype detection (myeloid neoplasms) detection using target enrichment and TPM/CNV data as described herein.
**FIG.15** is an exemplary graph depicting chromosomal translocations of a patient with acute lymphoblastic leukemia using RNA sequencing from cfRNA as described herein.
**FIG.16** is an exemplary graph depicting chromosomal translocations of a patient with acute myeloid leukemia using RNA sequencing from cfRNA as described herein.
**FIG.17** is an exemplary graph depicting chromosomal structural abnormalities in a pediatric patient with acute lymphoblastic leukemia using standard approaches like CNVkit approach.
**FIG.18** is another exemplary graph depicting chromosomal structural abnormalities in a pediatric patient with acute lymphoblastic leukemia using standard approaches like CNVkit approach.
**FIG.19** is an exemplary graph depicting prediction of the presence of a cancer specific mutation in circulation (recurrence/minimal residual disease) using cfRNA.
**FIG.20** is an exemplary graph depicting prediction of the presence of a cancer specific mutation in circulation (recurrence/minimal residual disease) using cfTNA.

### Detailed Description

The invention is as defined by the claims, and any other aspects, configurations, or embodiments set forth herein not falling within the scope of the claims are for information only. Additionally, any references in the description to methods of treatment refer to the compounds, pharmaceutical compositions, and medicaments of the present invention for use in a method of treatment of the human (or animal) body by therapy (of for diagnosis). The inventor has now discovered that numerous difficulties associated with analysis of cell-free nucleic acids isolated from a biological fluid such as blood can be overcome using systems and methods in which cfTNA and cfRNA and fragments thereof are isolated from the same sample, and in which the so obtained samples are subjected to reverse transcription to generate respective cDNA libraries. To improve analysis even further, the cDNA libraries are then subjected to target enrichment using (hyper)tiled hybridization probes prior to amplification, NGS sequencing, and *in silico* analysis.

Notably, the systems and methods presented herein not only avoid loss of nucleic acids as compared to currently known methods, but also provide superior detection of mutations with remarkable sensitivity and specificity. Indeed, it should be appreciated that an overwhelming majority (if not substantially all) of the circulating nucleic acids encoding genes of interest can be surveyed using the systems and methods presented herein, regardless of their physical integrity, copy number, and strength of expression. Consequently, sequencing data obtained by the methods presented herein provide not only a highly accurate and comprehensive representation of circulating nucleic acids, but also enable machine learning to generate trained models that can be used with high confidence (*e.g.,* AUC ≥ 0.7, and more typically AUC ≥ 0.8) to identify a cancer, a type of cancer, minimal residual disease, etc. Similarly, the systems and methods presented herein also allow to identify cancer sub-types with high confidence.

For example, in one typical process, the biological fluid is peripheral blood collected in EDTA containing blood collection tubes, and a plasma fraction is prepared from the blood via centrifugation as is well known in the art. Total nucleic acid (cfTNA) is then extracted from the plasma sample using silica-based beads suitable for recovery of DNA having a size of at least 50 base pairs and RNA having a size of at least 17 nucleotides. In this context it should be noted that the so recovered nucleic acids will include full-length genes and transcripts as well as all fragments thereof, even where such fragments are very small *(e.g.,* <150 bp/nt, or <100 bp/nt, or <75 base bp/nt, and even smaller). At least some of the so isolated cfTNA is then split into two portions, and one of the two portions is subjected to DNAse treatment yielding corresponding cfRNA. Advantageously, this step enriches the sample in RNA relative to the DNA and can so serve as an independent but corresponding sample (The DNA/RNA quantities in the untreated cfTNA sample are typically between 80%/20% and 95%/5%). Thus, it should be recognized that two distinct samples (cfTNA and cfRNA) are generated from the same biological fluid.

Each of the two distinct samples is then subjected to reverse transcription after optional rRNA depletion by first strand synthesis (typically with small random primers), second strand synthesis (which may be performed using dUTP for strand specificity), and A-tailing. The so obtained first and second cDNA libraries are then ligated to 3'-dTMP adapters. At this point, it should be noted that the cDNA library that is prepared from the cfTNA also contains cfDNA to which adapters are also ligated. Both first and second cDNA libraries are amplified using PCR and each amplification reaction is cleaned up for further processing. As will be readily appreciated, multiple samples can be combined for multiplexing where suitable adapters were employed as described in more detail below.

The so amplified first and second cDNA libraries are then subjected to target gene enrichment using multiple tiled hybridization probes for each target gene. Most typically, the entire target gene or transcript is targeted by hybridization probes having a step length of between 1 and 10 (*i.e.,* first and second hybridization probes bind to the target sequence at a linear distance of between 1-10 nt). It is further preferred that the hybridization probes will have a length of between 100-150 nt. In the present example, the target genes are genes encoding one or more cancer associated genes, cell signaling associated genes, immunophenotype associated genes, and/or receptor associated genes, and an exemplary collection of 1458 target genes is shown in **Table 1** below. Hybridization is performed in liquid phase over at least 8 hours and captured cDNA will be removed using magnetic beads.

Isolation of the target nucleic acids yields first and second target-enriched cDNA libraries that are then subjected to a further amplification (typically between 6-15 amplification cycles), and the so amplified target-enriched cDNA libraries are then sequenced using NGS sequencing (typically paired-end sequencing). Upon conclusion of the sequencing, the data for the first and second target enriched cDNA libraries are processed for deconvolution, mutant and fusion calls, expression level determination, identification of CNV/SNP variants, and determination of allele fraction and genomic rearrangements. Moreover, and as is also shown in more detail below, some or all of the data of the first and/or second target enriched cDNA libraries can be used to produce trained models and/or used in one or more trained models to identify the presence of a cancer, to classify or even sub-type the cancer, detect residual disease, and to detect cytogenetic changes (e.g., translocation, copy number changes, etc.).

With respect to suitable biological fluids it should be appreciated that numerous biological fluids other than whole blood, plasma, and serum are also deemed appropriate for use herein, and suitable fluids include all fluids that can or are suspected to contain cell free nucleic acids. As will also be readily appreciated, the biological fluid can be obtained from any suitable source, and especially from a human or a non-human mammal (livestock, companion animal, etc.). Moreover, it should be noted that the human or other mammal may be healthy or diagnosed with or suspected to have a condition or disease, particularly where such disease can be linked or attributed to a mutation in and/or (over- or under-)expression pattern of one or more genes. Therefore, the subject may be treatment naïve or undergoing treatment when the cfRNA and cfTNA is obtained from the subject. Viewed from a different perspective, use of the cfRNA and cfTNA is particularly beneficial for detection of a disease, monitoring the progression of a disease, monitoring the treatment effect of a treatment given to treat the disease, as well as for detection of residual or recurring disease.

Therefore, contemplated fluids include saliva, urine, synovial fluid, cerebrospinal fluid, cyst fluid (*e.g.*, pancreatic cyst) and ascites fluid. Consequently, and depending on the type of biological fluid, it should be noted that numerous known manners of isolation of the cfRNA and cfTNA are contemplated, including isolation via adsorption onto a solid carrier (e.g., silica or amine modified carrier), non-covalent binding to polybasic materials (and especially proteins), electrophoretic or other electrochemical separation, microfluidic separation, etc. However, particularly preferred methods of isolation of cfRNA and cfTNA include those that use solid phase adsorption.

In addition, it should also be appreciated that the samples for the methods and systems presented herein need not necessarily be limited to fluids, but it should be recognized that such systems and methods can be used in conjunction with any sample that has a low content of nucleic acids, and where such nucleic acids may have undergone at least some degradation. Therefore, further contemplated samples include biopsy specimen (e.g., needle core, smear, brush, etc., which may be raw or processed), tissue slides (FFPE fixed or unfixed), minimal or residual forensic tissue samples, samples from ancient tissue (e.g.,>100 years of age), etc.

Regardless of the manner of isolation, it should be appreciated that the isolated cfRNA and cfDNA will not only represent full-length nucleic acids (with respect to a specific target gene or transcript) but also fragments thereof having lengths to a varying degree. Indeed, due to the particular source material for the cfTNA and cfRNA, it is expected that the isolated material will predominantly (e.g., at least 50%, or at least 60%, or at least 70%, or at least 75%, or at least 80%, or at least 85%, or at least 90%) comprise fragments of a plurality of target genes and transcripts thereof. Therefore, it is contemplated that the majority of the plurality of target genes and transcripts will have a length of equal of less than 1,000 bp/nt, or equal of less than 900 bp/nt, or equal of less than 800 bp/nt, or equal of less than 700 bp/nt, or equal of less than 600 bp/nt, or equal of less than 500 bp/nt, or equal of less than 400 bp/nt, or equal of less than 300 bp/nt, and even less.

Viewed from a different perspective, at least some of the cfRNA isolated using the procedures contemplated herein may have a length range of between 15-50 nt, or between 20-75 nt, or between 17-100 nt, or between 20-150 nt, or between 20-200 nt, or between 50-300 nt. Similarly, at least some of the cfDNA present in the cfTNA isolated using the procedures contemplated herein may have a length range of between 50-100 bp, or between 75-150 bp, or between 75-200 bp, or between 100-300 bp, or between 50-350 bp. Therefore, the overall size distribution of the cfRNA and cfTNA may have a peak at a length between 100-200 bp/nt, or between 150-250 bp/nt, or between 200-300 bp/nt, typically at a length distribution width (covering 90% of all isolated nucleic acids) of between 50-400 bp/nt or between 75-500 bp/nt.

In still further contemplated aspects, it should be appreciated that while it is generally preferred that the cfRNA fraction is prepared from a parent volume of a cfTNA isolation, the cfRNA fraction may also be prepared separately from the cfTNA from the same sample, either using methods and materials designed to selectively isolate cfRNA only, or from a second and different volume of the sample. Alternatively, cfRNA and cfDNA may be separately isolated form the same biological fluid and a cfTNA fraction may be reconstituted from various proportions of isolated cfRNA and cfDNA (e.g., about 5-15% cfRNA and 85-95% cfDNA, or about 15-25% cfRNA and 75-85% cfDNA, or about 30-50% cfRNA and 50-70% cfDNA).

As will be readily appreciated, reverse transcription of the isolated cfRNA molecules in the cfRNA and cfTNA samples can follow all standard protocols known in the art. In addition, it should be appreciated that the cfRNA and cfTNA samples may be pre-processed to remove ribosomal RNA. Moreover, where desirable, the cfRNA and cfTNA samples may also be subjected to size fragmentation using thermal treatment in the presence of magnesium, or shearing, and/or ultrasonication to produce a population of fragmented molecules having an average size of, for example, between 200 and 400 base pairs/nucleotides. Most typically, reverse transcription will make use of universal primers, especially for first strand synthesis. Second strand synthesis can also follow established procedures and may include use of oligo-T primers, random primers, and/or targeted second strand primers (e.g., using sequences from a target enrichment list). Likewise, it is contemplated that the second strand synthesis may be strand-specific using dUTP incorporation. Regardless of the manner of cDNA generation, it is preferred that the so generated cDNA libraries are subjected to A-tailing (addition of single adenosine) that facilitates adapter ligation to the cDNA library members (typically using dsDNA adapter with 3'-dTMP overhang to allow ligation to the A-tailed library members).

Likewise, it should be recognized that the choice of adapters is not limiting to the inventive subject matter presented herein, and that the choice of adapter will typically be driven by the specific manner of downstream processing. For example, where the downstream processing uses Illumina-type next generation sequencing, adapters will typically include sequence portions that will specifically bind to complementary sequences on a flow cell or lane to allow for cluster formation. Among other such sequence portions, p5 and p7 sequence portions are especially deemed suitable for use herein. Moreover, and particularly where samples are multiplexed, contemplated adapters may also include unique first and/or second index portions that allow for post-sequencing deconvolution. As will also be readily recognized, the adapters will typically include appropriate sequencing primer binding site sequence portion to so enable paired-end sequencing. However, it further contemplated aspects, various alternative adaptors or even no adaptors may be used, especially where the sequencing is not paired end sequencing (e.g., nanopore sequencing, single molecule real time sequencing, ion torrent sequencing, SOLiD sequencing, etc.) The so obtained first and second cDNA libraries can then be amplified and/or enriched for a desired set of target genes. At this point, it should be noted that as the first and second cDNA libraries were prepared from the same biological fluid (and most typically from the same cfTNA isolation) these two cDNA libraries represent two distinct but complementary views of the same sample: one enriched in RNA (relative to DNA) and another rich in DNA (relative to RNA).

With respect to target enrichment it is contemplated that the first and second cDNA libraries (preferably after adapter ligation) are subjected to target enrichment to enrich the libraries with a selection of genes of interest. Most typically, the genes of interest will be associated with a disease or a condition but may also be selected on the basis of general health status or age or other non-health related status. For example, disease related genes of interest will typically include one or more genes that are associated with or causative for a particular disease. Among other things, where the disease is cancer, the cancer related genes may be indicative of the presence of a cancer, the type of cancer, a recurrence of cancer, and/or or residual cancer post treatment. Therefore, particularly contemplated target genes include cell signaling associated genes *(e.g.,* to identify the presence or quantity of a cell surface receptor), checkpoint inhibition related genes *(e.g.,* to identify the immune status of a cancer), genes encoding cell surface enzymes, genes associated with an immunophenotype (e.g., to identify presence or quantity of a cell surface receptor and/or presence or quantity of a cell surface enzyme), and/or genes encoding one or more cell surface receptors. Moreover, cancer specific genes may also include those that encode specific mutant forms of a known gene *(e.g.,* fusion products of kinases, truncated forms of cell surface receptors or signaling components), and mutant forms that are specific to a neoplasm and patient (*i.e.,* tumor- and patient specific neoantigens). Therefore, it should be appreciated that the gene selected for enrichment may be used to identify the presence of a cancer, classify a specific cancer, determine a clinical course or response to a therapy, or identify relapse of the disease.

Moreover, it should be appreciated that the methods presented herein are not only useful to identify mutations in a gene of a cancer (or other diseased cell) but that expression levels of mutated and non-mutated genes can be determined, adding a further dimension of clinical information suitable for identification and treatment of a disease. For example, such added information is particularly beneficial in cases where the sole identification of a mutated gene may be clinically irrelevant as a pharmaceutical target where that mutated gene is only weakly or not at all expressed.

In addition, it should be recognized that contemplated systems and methods presented herein not only make use of circulating nucleic acid degradation products and fragments having relatively small size (e.g., between 17-50 RNA nucleotides and/or 50-300 DNA base pairs), but specifically enrich these fragments using tiled or even hyper-tiled target enrichment to thereby maximize capture of all variants present in the cell free biological fluid. For example, in some embodiments, each target gene is targeted by a plurality of hybridization probes that bind to the target cDNA in a tiled (partially overlapping) fashion with a step length (*i.e.*, linear distance of 3'-ends of first and second hybridization probes when bound to the target gene and expressed in bases) of n, wherein n is an integer between 1-5. In other embodiments, n is between 5-10, or between 10-15, or between 15-20, or between 20-30, or between 30-50, or between 50-70, or between 70-100. Therefore, and viewed from a different perspective, the plurality of hybridization probes will provide a tiling density of at least 2, or at least 3, or at least 4, or at least 5, or at least 6, or at least 7, or at least 8, or at least 9, or at least 10, or between 10-20, or between 20-40, or between 40-60, and even higher where longer hybridization probes are being used. Consequently, it should be recognized that the linear length of the hybridization probes suitable for use herein may be between 20-40 bases, or between 40-70 bases, or between 70-100 bases, or between 100-150 bases, and even longer. Thus, the hybridization probes will cover the entire length of each target gene in a large multiplicity of positions. Of course, it should be noted that the hybridization probes will typically comprise a moiety that allows physical separation of the hybridization probes with the bound target to so facilitate target enrichment, and suitable moieties include magnetic beads, color-coded beads, affinity agents

### (e.g., biotin, avidin, his-tag, cellulose binding protein, etc.)

Most preferably, the hybridization probes will be combined with the cDNA libraries in a liquid phase for a time sufficient to allow for sequence specific annealing. As will be readily appreciated, longer hybridization probes will require a longer period of time to specifically and completely anneal. Consequently, target capture by the hybridization probes may be in the range of between 2-4 hours, or between 4-8 hours, or between 8-12 hours, and in some cases even longer. Regardless of the type of captured cDNA, the hybrid formed between the hybridization probe and the captured cDNA is removed from the remainder of the unbound cDNA library members. In this context it should be recognized that the so enriched target nucleic acids will include cfDNA molecules and cDNA molecules (from reverse transcription of the cfRNA). In addition, it should be appreciated that the so isolated enriched target nucleic acids represent not only full-length RNA molecules of the cfTNA and cfRNA fraction, but also all fragments and degradation products originally present in the biological fluid. As such, capture of the circulating nucleic acids will provide a significantly improved representation of the cell free nucleic acids as released from the diseased cells. Indeed, it is estimated that the first and/or second target enriched cDNA libraries represent at least 80%, or at least 85%, or at least 90%, or at least 92%, or at least 94%, or at least 96%, or at least 98% of all nucleic acids present in the biological fluid that correspond to the target cDNA.

To facilitate sequencing, the first and second target enriched cDNA libraries are subjected to target specific amplification. As will be readily appreciated, such amplification can advantageously use the anchoring, sequencing, and/or index sequence portions of the adapter (which beneficially reduces amplification bias due to target specific sequences). Most typically, amplification of the first and second target enriched cDNA libraries will run through 6-15 amplification cycles to provide sufficient material for sequencing, archiving, and repeat analyses. As already noted earlier, it should be appreciated that the particular manner of sequencing is not limiting to the inventive subject matter. However, it is generally preferred that the sequencing is performed using a next generation (e.g., paired-end) sequencing or other high-throughput method. Sequencing of the first and second target enriched cDNA libraries will preferably be performed to a depth of at least 10x, or at least 20x, or at least 30x, or at least 40x, or at least 50x, or at least 100x, and even more where desired.

Regardless of the method of sequencing, it should be appreciated that two data sets are obtained from the amplified target enriched first and second cDNA libraries that will provide distinct albeit complementary information as is also discussed in more detail below. Advantageously, the inventor discovered that use of the systems and methods presented herein allowed for identification and quantification of a large variety of mutants, alternate transcripts, and poorly or non-expressed mutations in genes, as well as for detection of mutations leading to high instability in a RNA transcript as is also shown in more detail below. In addition, the systems and methods presented herein also enable quantification of the expression level of a (mutated) target gene using the cfRNA fraction, which can be further contextualized with copy number variation information obtained from the cfTNA fraction. Similarly, contemplated systems and methods allow for improved analysis of allele fractions where both cfTNA and cfRNA fractions are analyzed.

Thus, use of first and second target-enriched cDNA libraries significantly increases sensitivity of mutant (*e.g.*, SNV, indel, translocation) detection. Among other things, RNA converted to cDNA generated from each cell is more abundant that DNA generated from each cell. Therefore, and as is shown in more detail below, the co-sequencing of DNA in the TNA sequencing will compensate for detecting mutations in cases where the RNA is degraded, for example, due to change in its stability on account of a mutation. Indeed, it should be recognized that the data obtained from the cfTNA and cfRNA fraction are now sufficient to generate via machine learning trained models that enable identification and even prediction of diseases, disease states, and disease conditions with high confidence as is shown in more detail below. Moreover, the so obtained information based on the cfTNA and cfRNA fraction can also be used to predict an immunophenotype and/or an immunohistochemical profile. As is also discussed in more detail below, the so obtained information based on the cfTNA and cfRNA fraction can also be used to perform a virtual cytogenetic analysis.

### Examples

Nucleic acid extraction (general protocol): Unless specified otherwise, all nucleic acid extraction was from whole peripheral blood collected in EDTA vacutainer tubes. After separation of plasma from cell components, 1 ml plasma was used.

To capture small fragmented RNA and TNA, the inventor adapted a method originally designed for capturing microRNA in circulation. In the examples below, the inventor used a commercially available kit (Apostle MiniMax High Efficiency cfRNA/cfDNA isolation kit) and followed the manufacturer's protocol. After isolation of the cfRNA/cfDNA, half of the cfTNA sample was treated with DNase to obtain a cfRNA sample, while the other half was maintained unchanged. Each subject's cfTNA and cfRNA samples were then processed in parallel to produce respective cDNA libraries for each subject. Reverse transcription and adapter ligation was performed using a commercially available kit (KAPA RNA HyperPrep kit) following the manufacturer's instructions. Reverse transcription and adapter ligation included the following steps: 1st strand synthesis using random hexamer primers followed by second strand synthesis using KAPA RNA HyperPrep Kit primers, and A-tailing. Upon completion of A-tailing, Illumina NGS adapters with index sequence portions were ligated to the cfDNA and cDNA and the first and second libraries were amplified using KAPA RNA HyperPrep Kit primers for 14 cycles. In this context it should be appreciated that the second strand synthesis makes preferably use of the same oligonucleotides that are being used in the downstream target enrichment as is discussed in more detail below, thereby greatly increasing sensitivity and specificity.

Amplification reactions were then cleaned up using KingFisherFlex clean up system and the amplified first and second libraries were quantified. 8-plex DNA sample library pools were prepared from the subjects' libraries by Janus for hybridization with target specific hybridization probes ('Target Enrichment Probes'). The probes were GTC-designed KAPA Target Enrichment Probes covering a total of 1458 genes (as listed in **Table 1**) for hybridization overnight (at least 8 hours). The Target Enrichment Probes for each gene in the target genes of Table 1 had a length of 60 nucleotides (and thus provided a step length of between 1-60; the particular step lengths will be dictated by primer design software), resulting in a tiling density of between 2-59. After target hybridization, KAPA beads were used to capture the multiplexed DNA libraries, and each library was amplified to so obtain first and second target-enriched cDNA libraries. The first and second target-enriched cDNA libraries were then cleaned up and checked using an Agilent TapeStation analyzer. Each library was then normalized, pooled, denatured, and loaded onto a Novaseq 6000 sequencer for sequencing using pair-end 100x2 cycles.

**Table 1**

| | | | | | | | |
|---|---|---|---|---|---|---|---|
| ABCC3 | ABI1 | ABL1 | ABL2 | ABLIM1 | ACACA | ACE | ACER1 |
| ACKR3 | ACP3 | ACSBG1 | ACSL3 | ACSL6 | ACVR1B | ACVR1C | |
| ACVR2A | ADD3 | ADGRA2 | ADGRG7 | ADM | AFDN | AFF1 | AFF3 |
| AFF4 | AFP | AGR3 | AHCYL1 | AHI1 | AHR | AIP | |
| AK2 | AK5 | AKAP12 | AKAP6 | AKAP9 | AKR1C3 | AKT1 | AKT2 |
| AKT3 | ALDH1A1 | ALDH2 | ALDOC | ALK | AMER1 | AMH | |
| ANGPT1 | ANKRD28 | ANLN | ANPEP | APC | APH1A | APLP2 | APOD |
| AR | ARAF | ARFRP1 | ARG1 | ARHGAP20 | ARHGAP26 | ARHGEF12 | |
| ARHGEF7 | ARID1A | ARID2 | ARIH2 | ARNT | ARRDC4 | ASMTL | ASPH |
| ASPSCR1 | ASTN2 | ASXL1 | ATF1 | ATF3 | ATG13 | ATG5 | |
| ATIC | ATL1 | ATM | ATP1B4 | "ATP6V1G2-DDX39B, pseudo" | ATP8A2 | ATR | ATRNL1 |
| ATRX | AURKA | AURKB | AUTS2 | AXIN1 | AXL | B2M | |
| B3GAT1 | BACH1 | BACH2 | BAG4 | BAIAP2L1 | BAP1 | BARD1 | BAX |
| BAZ2A | BCAS3 | BCAS4 | BCL10 | BCL11A | BCL11B | BCL2 | |
| BCL2A1 | BCL2L1 | BCL2L2 | BCL3 | BCL6 | BCL7A | BCL9 | BCOR |
| BCORL1 | BCR | BDNF | BHLHE22 | BICC1 | BIN1 | BIRC3 | |
| BIRC6 | BLM | BMP4 | BMPR1A | BRAF | BRCA1 | BRCA2 | BRD1 |
| BRD3 | BRD4 | BRIP1 | BRSK1 | BRWD3 | BTBD18 | BTG1 | |
| BTG2 | BTK | BTLA | BUB1B | C10orf55 | C11orf1 | C11orf54 | C11orf95 |
| C2CD2L | CACNA1F | CACNA1G | CACNA2D3 | CAD | CALR | CAMK2A | |
| CAMK2B | CAMK2G | CAMTA1 | CANT1 | CAPRIN1 | CAPZB | CARD 11 | CARM1 |
| CARMIL2 | CARS1 | CASP3 | CASP7 | CASP8 | CAV1 | CBFA2T3 | |
| CBFB | CBL | CBLB | CBLC | CCAR2 | CCDC28A | CCDC6 | CCDC88C |
| CCK | CCL2 | CCNA2 | CCNB1IP1 | CCNB3 | CCND1 | CCND2 | |
| CCND3 | CCNE1 | CCNG1 | CCT6B | CD14 | CD19 | CD1A | CD2 |
| CD200 | CD22 | CD24 | CD247 | CD274 | CD28 | CD33 | |
| CD34 | CD36 | CD38 | CD3D | CD3E | CD3G | CD4 | CD40 |
| CD44 | CD47 | CD5 | CD52 | CD58 | CD59 | CD68 | |
| CD7 | CD70 | CD74 | CD79A | CD79B | CD81 | CD8A | CD8B |
| CD9 | CDC14A | CDC14B | CDC25A | CDC25C | CDC42 | CDC73 | |
| CDH1 | CDH11 | CDK1 | CDK12 | CDK2 | CDK4 | CDK5RAP2 | CDK6 |
| CDK7 | CDK8 | CDK9 | CDKL5 | CDKN1A | CDKN1B | CDKN1C | |
| CDKN2A | CDKN2B | CDKN2C | CDKN2D | CDX1 | CDX2 | CEACAM8 | CEBPA |
| CEBPB | CEBPD | CEBPE | CENPF | CENPU | CEP170B | CEP57 | |
| CEP85L | CHCHD7 | CHD2 | CHD6 | CHEK1 | CHEK2 | CHIC2 | CHL1 |
| CHMP2B | CHN1 | CHST11 | CHUK | CIC | CIITA | CILK1 | |
| CIP2A | CIT | CKB | CKS1B | CLP1 | CLTA | CLTC | CLTCL1 |
| CMKLR1 | CNBP | CNOT2 | CNTN1 | CNTRL | COG5 | COL11A1 | |
| COL1A1 | COL1A2 | COL3A1 | COL6A3 | COL9A3 | COMMD1 | COX6C | CPNE1 |
| CPS1 | CPSF6 | CRADD | CREB1 | CREB3L1 | CREB3L2 | CREBBP | |
| CRKL | CRLF2 | CRTC1 | CRTC3 | CSF1 | CSF1R | CSF3 | CSF3R |
| CSNK1G2 | CSNK2A1 | CTCF | CTDSP2 | CTLA4 | CTNNA1 | CTNNB1 | |
| CTNND2 | CTRB1 | CTRB2 | CTSA | CUX1 | CXCL8 | CXCR4 | CXXC4 |
| CYFIP2 | CYLD | CYP1B1 | CYP2C19 | DAB2IP | DACH1 | DACH2 | |
| DAXX | DCLK2 | DCN | DDB2 | DDIT3 | DDR2 | DDX10 | DDX20 |
| DDX39B | DDX3X | DDX41 | DDX5 | DDX6 | DEK | DGKB | |
| DGKI | DGKZ | DICER1 | DIRAS3 | DIS3L2 | DKK1 | DKK2 | DKK4 |
| DLEC1 | DLL1 | DLL3 | DLL4 | DMRT1 | DMRTA2 | DNAJB1 | |
| DNM1 | DNM2 | DNM3 | DNMT1 | DNMT3A | DNTT(DTT) | DOCK1 | DOT1L |
| DPM1 | DPP4 | DPYD | DST | DTX1 | DTX4 | DUSP2 | |
| DUSP22 | DUSP26 | DUSP9 | E2F1 | EBF1 | ECT2L | EDIL3 | EDNRB |
| EED | EEFSEC | EGF | EGFR | EGR1 | EGR2 | EGR3 | |
| EGR4 | EIF4A2 | EIF4E | ELF4 | ELK4 | ELL | ELN | ELOVL2 |
| ELP2 | EML1 | EML4 | EMSY | ENG | ENPP2 | EP300 | |
| EP400 | EPC1 | EPCAM | EPHA10 | EPHA2 | EPHA3 | EPHA5 | EPHA7 |
| EPHB1 | EPHB6 | EPO | EPOR | EPS15 | ERBB2 | ERBB3 | |
| ERBB4 | ERC1 | ERCC1 | ERCC2 | ERCC3 | ERCC4 | ERCC5 | ERCC6 |
| ERG (prostate) | ERLIN2 | ESR1 | ETS1 | ETS2 | ETV1 | ETV4 | |
| ETV5 | ETV6 | EWSR1 | EXOSC6 | EXT1 | EXT2 | EYA1 | EYA2 |
| EZH2 | EZR | FAF1 | FANCA | FANCB | FANCC | FANCD2 | |
| FANCE | FANCF | FANCG | FANCI | FANCL | FANCM | FAS | FASLG |
| FBN2 | FBXO11 | FBXO31 | FBXW7 | FCER2 | FCGBP | FCGR1A(CD64) | |
| FCGR2B (CD32) | FCGR3A (CD16) | FCRL4 | FEN1 | FEV | FGF1 | FGF10 | FGF13 |
| FGF14 | FGF19 | FGF2 | FGF23 | FGF3 | FGF4 | FGF6 | |
| FGF8 | FGF9 | FGFR1 | FGFR1OP2 | FGFR2 | FGFR3 | FGFR4 | FH |
| FHIT | FHL2 | FIP1L1 | FLCN | FLI1 | FLNA | FLNC | |
| FLT1 | FLT3 | FLT3LG | FLT4 | FLYWCH1 | FNBP1 | FOS | FOSB |
| FOSL1 | FOXL2 | FOXO1 | FOXO3 | FOXO4 | FOXP1 | FOXP3 | |
| FRK | FRMPD4 | FRS2 | FRYL | FSTL3 | FUS | FUT1 | FUT4(CD15) |
| FZD10 | FZD2 | FZD3 | FZD6 | FZD7 | FZD8 | GAB1 | |
| GABRG2 | GADD45B | GANAB | GAS1 | GAS7 | GATA1 | GATA2 | GATA3 |
| GATA6 | GBP2 | GDF6 | GFAP | GHR | GID4 | GIT2 | |
| GLI1 | GLI3 | GMPS | GNA11 | GNA12 | GNA13 | GNAI1 | GNAQ |
| GNAS | GNG4 | GOLGA5 | GOPC | GOSR1 | GOT1 | GPC3 | |
| GPHN | GPR34 | GRB10 | GRB2 | GRHPR | GRID1 | GRIN2A | GRIN2B |
| GRM1 | GRM3 | GSK3B | GSN | GTF2I | GTSE1 | GYPA(CD235a) | |
| H1-2 | H1-3 | H1-4 | H2AC11 | H2AC16 | H2AC17 | H2AC6 | H2AX |
| H2BC11 | H2BC12 | H2BC17 | H2BC4 | H2BC5 | H3-3A | H3C2 | |
| H4C9 | HAS2 | HDAC1 | HDAC2 | HDAC3 | HDAC4 | HDAC5 | HDAC6 |
| HDAC7 | HECW1 | HEPH | HERPUD1 | HES1 | HESS | HEY1 | |
| HGF | HHEX | HIF1A | HIP1 | HIPK1 | HIPK2 | HLA-DRA | HLA-DRB1 |
| HLF | HMGA1 | HMGA2 | HMGB1 | HNF1A | HNRNPA2B1 | HOOK3 | |
| HOXA10 | HOXA11 | HOXA13 | HOXA3 | HOXA9 | HOXC11 | HOXC13 | HOXD11 |
| HOXD13 | HOXD9 | HRAS | HSP90AA1 | HSP90AB1 | HSPA1A | HSPA1B | |
| HSPA2 | HSPA4 | HSPA5 | HTRA1 | HUWE1 | IBSP | ICAM1 | ID1 |
| ID3 | ID4 | IDH1 | IDH2 | IFNG | IFRD1 | IGF1 | |
| IGF1R | IGFBP2 | IGFBP3 | IKBKB | IKBKE | IKZF1 | IKZF2 | IKZF3 |
| IL12RB2 | IL13 | IL13RA2 | IL15 | IL1B | IL1R1 | IL1RAP | |
| IL2 | IL21R | IL2RA | IL3 | IL3RA( CD123) | IL6 | IL7R | INHBA |
| INPP4A | INPP4B | INPP5A | INPP5D | IQCG | IRAG2 | IRF1 | |
| IRF2BP2 | IRF4 | IRF8 | IRS1 | IRS2 | IRS4 | ITGA2B(CD41) | ITGA5(CD49e) |
| ITGA7 | ITGA8 | ITGAE(CD103) | ITGAM(CD11B) | ITGAV (CD51) | ITGAX(CD11C) | ITGB3(CD61) | |
| ITGB4(CD104) | ITK | ITPKA | JAG2 | JAK1 | JAK2 | JAK3 | JARID2 |
| JAZF1 | JUN | KALRN | KAT6A | KAT6B | KCNB1 | KDM1A | |
| KDM2B | KDM4C | KDM5A | KDM5C | KDM6A | KDR | KDSR | KEAP1 |
| KIAA0232 | KIAA1549 | KIF5B | KIT(CD117) | KLF4 | KLHL6 | KLK2(prostate) | |
| KLK3 | KLK7 | KLRC1 | KMT2A | KMT2B | KMT2C | KMT2D | KNL1 |
| KPNB1 | KRAS | KRT1 | KRT10 | KRT16 | KRT17 | KRT19 | |
| KRT2 | KRT5 | KRT6A | KRT6B | KRT8 | KSR1 | KTN1 | LAMA1 |
| LAMAS | LAMP1 | LAMP2 | LASP1 | LCK (T cell) | LCP1 | LEF1 (T cell/CLL) | |
| LEFTY2 | LFNG | LGALS3 | LGR5 | LHFPL3 | LHFPL6 | LHX2 | LHX4 |
| LIFR (CD118) | LILRA4 | LINGO2 | LMBRD1 | LMO1 | LMO2 | LMO7 | |
| LNP1 | LOX | LPAR1 | LPP | LPXN | LRIG3 | LRP1B | LRP5 |
| LRPPRC | LRRC37B | LRRC59 | LRRC7 | LRRK2 | LTBP1 | LYL1 | |
| LYN | MACROD1 | MAD2L1 | MADD | MAF | MAFB | MAGED1 | MAGEE1 |
| MALT1 | MAML1 | MAML2 | MAP2 | MAP2K1 | MAP2K2 | MAP2K3 | |
| MAP2K4 | MAP2K5 | MAP2K6 | MAP2K7 | MAP3K1 | MAP3K14 | MAP3K6 | MAP3K7 |
| MAPK1 | MAPK3 | MAPK8 | MAPK8IP2 | MAPK9 | MAPRE1 | MATK | |
| MAX | MB21D2 | MBNL1 | MBTD1 | MCAM | MCL1 | MDC1 | MDH1 |
| MDM2 | MDM4 | MEAF6 | MECOM | MED12 | MEF2B | MEF2C | |
| MEF2D | MELK | MEN1 | MET | METTL18 | METTL7B | MFNG | MGMT |
| MIB1 | MIPOL1 | MITF | MKI67 | MLANA | MLF1 | MLH1 | |
| MLLT1 | MLLT10 | MLLT11 | MLLT3 | MLLT6 | MME(CD10) | MMP7 | MMP9 |
| MN1 | MNAT1 | MNX1 | MPL | MPO | MRE11 | MRTFA | |
| MRTFB | MS4A1 (CD20) | MSH2 | MSH3 | MSH6 | MSI2 | MSN | MTCP1 |
| MTOR | MTUS2 | MUC1 | MUC16 | MUTYH | MYB | MYBL1 | |
| MYC | MYCL | MYCN | MYD88 | MYH11 | MYH9 | MYO18A | MYO1F |
| NAB2 | NACA | NAPA | NAPSA | NAV3 | NBN | NBR1 | |
| NCAM1 | NCKIPSD | NCOA1 | NCOA2 | NCOA3 | NCOA4 | NCOR2 | NCSTN |
| NDC80 | NDE1 | NDRG1 | NDUFAF1 | NEDD4 | NEURL1 | NF1 | |
| NF2 | NFATC1 | NFATC2 | NFE2L2 | NFIB | NFKB1 | NFKB2 | NFKBIA |
| NGF | NGFR | NIN | NIPBL | NKX2-1 | NKX2-5 | NKX3-1 | |
| NOD1 | NODAL | NONO | NOS3 | NOTCH1 | NOTCH2 | NOTCH3 | NOTCH4 |
| NPM1 | NPM2 | NR3C1 | NR4A3 | NR5A1 | NR6A1 | NRAS | |
| NRG1 | NSD1 | NSD2 | NSD3 | NT5C2 | NTF3 | NTF4 | NTRK1 |
| NTRK2 | NTRK3 | NUMA1 | NUP107 | NUP214 | NUP93 | NUP98 | |
| NUTM1 | NUTM2A | NUTM2B | OFD1 | OGA | OLIG1 | OLIG2 | OLR1 |
| OMD | P2RY8 | PAFAH1B2 | PAG1 | PAK1 | PAK3 | PAK5 | |
| PAK6 | PALB2 | PAPPA | PASK | PATZ1 | PAX3 | PAX5 | PAX7 |
| PAX8 | PBRM1 | PBX1 | PC | PCA3 | PCBP1 | PCLO | |
| PCM1 | PCNA | PCSK7 | "PDCD1 (PD-1, CD279)" | PDCD11 (ALG4) | PDCD1LG2 (PD-L2) | PDE4DIP | PDGFA |
| PDGFB | PDGFD | PDGFRA | PDGFRB | PDK1 | PEG3 | PER1 | |
| PFDN5 | PHB | PHF1 | PHF23 | PHF6 | PHOX2B | PI4KA | PICALM |
| PIK3CA | PIK3CB | PIK3CD | PIK3CG | PIK3R1 | PIK3R2 | PIM1 | |
| PIMREG | PKM | PLA2G2A | PLA2GS | PLAG1 | PLAT | PLAU | PLCB1 |
| PLCB4 | PLCG1 | PLCG2 | PLEKHM2 | PLPP3 | PML | PMS1 | |
| PMS2 | POFUT1 | POLD1 | POLD4 | POLR2H | POM121 | POMGNT1 | POSTN |
| POT1 | POU2AF1 | POUSF1 | PPARG | PPARGC1A | PPFIA2 | PPFIBP1 | |
| PPM1D | PPP1CB | PPP1R13B | PPP1R13L | PPP2CB | PPP2R1A | PPP2R1B | PPP2R2B |
| PPP3CA | PPP3CB | PPP3CC | PPP3R1 | PPP3R2 | PPP4C | PRCC | |
| PRDM1 | PRDM16 | PRDM7 | PRF1 | PRG2 | PRICKLE1 | PRKACA | PRKACG |
| PRKAR1A | PRKCA | PRKCB | PRKCD | PRKCG | PRKDC | PRKG2 | |
| PRMT1 | PRMT8 | PROM1 | PRRX1 | PRRX2 | PRSS8 | PSD3 | PSEN1 |
| PSIP1 | PSMD2 | PTBP1 | PTCH1 | PTCRA | PTEN | PTGS2 | |
| PTK2 | PTK2B | PTK7 | PTPA | PTPN11 | PTPN2 | PTPN6 | PTPRA |
| PTPRC(CD45) | PTPRK | PTPRO | PTPRR | PTTG1 | RABEP1 | RAC1 | |
| RAC2 | RAC3 | RAD21 | RAD50 | RAD51 | RAD51B | RAD51C | RAD51D |
| RAD52 | RAF1 | RALGDS | RANBP17 | RANBP2 | RAP1GDS1 | RARA | |
| RASAL1 | RASGEF1A | RASGRF1 | RASGRF2 | RASGRP1 | RB1 | RBM15 | RBM6 |
| RCHY1 | RCOR1 | RCSD1 | RECQL4 | REEP3 | REG3A | RELA | |
| RELN (glioma) | RERG | RET | RGS7 | RHBDF2 | RHOA | RHOD | RHOH |
| RICTOR | RMI2 | RNF213 | RNF43 | ROBO1 | ROBO2 | ROS1 | |
| RPA3 | RPL22 | RPN1 | RPN2 | RPS21 | RPS6KA1 | RPS6KA2 | RPS6KA3 |
| RPTOR | RREB1 | RRM1 | RRM2B | RTEL1 | RTEL1-TNFRSF6B | RTL8B | |
| RTN3 | RUNX1 | RUNX1T1 | RUNX2 | RYR3 | S1PR2 | SARNP | SATB2 |
| SBDS | SCGB2A2 | SCN8A | SDC1(CD138) | SDC4 | SDHA | SDHAF2 | |
| SDHB | SDHC | SDHD | SEC31A | SEPTIN2 | SEPTINS | SEPTIN6 | SEPTINS |
| SERP2 | SERPINE1 | SERPINF1 | SET | SETBP1 | SETD2 | SETD7 | |
| SF3B1 | SFPQ | SFRP2 | SFRP4 | SGK1 | SGPP2 | SH2D5 | SH3BP1 |
| SH3D19 | SH3GL1 | SH3GL2 | SHC1 | SHC2 | SHTN1 | SIK3 | |
| SIN3A | SIRT1 | SKP2 | SLC1A2 | SLC34A2 | SLC45A3 | SLC66A3 | SLC7A5 |
| SLCO1B3 | SLX4 | SMAD2 | SMAD3 | SMAD4 | SMAD6 | SMAP1 | |
| SMARCA1 | SMARCA4 | SMARCAS | SMARCB1 | SMC1A | SMC3 | SMO | SNAPC3 |
| SNCG | SNW1 | SNX29 | SNX9 | SOCS1 | SOCS2 | SOCS3 | |
| SOD2 | SORBS2 | SORT1 | SOS1 | SOX10 | SOX11 | SOX2 | SP1 |
| SP3 | SPECC1 | SPEN | SPN | SPOP | SPP1 | SPRY2 | |
| SPRY4 | SPTAN1 | SPTBN1 | SOSTM1 | SRC | SRF | SRGAP3 | SRRM3 |
| SRSF2 | SRSF3 | SS18 | SS18L1 | SSBP2 | SSX1 | SSX2 | |
| SSX2B | SSX4 | SSX4B | ST6GAL1 | STAG2 | STAT1 | STAT3 | STAT4 |
| STATSA | STATSB | STAT6 | STIL | STK11 | STRN | STX5 | |
| STYK1 | SUFU | SUGP2 | SULF1 | SUV39H2 | SUZ12 | SYK | SYP |
| TACC1 | TACC2 | TACC3 | TAF1 | TAF15 | TAFA2 | TAFA5 | |
| TAL1 | TAL2 | TAOK1 | TBL1XR1 | TBX15 | TCEA1 | TCF12 | TCF3 |
| TCF7L2 | TCL1A | TCTA | TEAD1 | TEAD2 | TEAD3 | TEAD4 | |
| TEC | TENM1 | TENT5C | TERF1 | TERF2 | TERT | TET1 | TET2 |
| TFDP1 | TFE3 | TFEB | TFG | TFPT | TFRC(CD71) | TG | |
| TGFB2 | TGFB3 | TGFBI | TGFBR2 | TGFBR3 | THADA | THBS1 | THRAP3 |
| TIAM1 | TIRAP | TLL2 | TLR4 | TLX1 | TLX3 | TMEM127 | |
| TMEM230 | TMEM30A | TMPRSS2 | TNC | TNF | TNFAIP3 | TNFRSF10B | TNFRSF10D |
| TNFRSF11A | TNFRSF14 (CD270) | TNFRSF17 (BCMA) | TNFRSF6B | TNFRSF8 (CD30) | TOP1 | TOP2A | |
| TOP2B | TP53 | TP53BP1 | TP63 | TP73 | TPD52L2 | TPM3 | TPM4 |
| TPO | TPR | TRAF2 | TRAF3 | TRAF5 | TRHDE | TRIM24 | |
| TRIM27 | TRIM33 | TRIP11 | TRPS1 | TSC1 | TSC2 | TSHR | TTF1 |
| TTK | TTL | TUSC3 | TYK2 | TYMS | U2AF1 | U2AF2 | |
| UBE2B | UBE2C | UFC1 | UFM1 | UPK3A | USP16 | USP42 | USP5 |
| USP6 | USP7 | UTP4 | VCAM1 | VEGFA | VEGFC | VEGFD | |
| VGLL3 | VHL | VTI1A | WASF2 | WDCP | WDFY3 | WDR1 | WDR18 |
| WDR70 | WDR90 | WEE1 | WIF1 | WNT10A | WNT10B | WNT11 | |
| WNT16 | WNT2B | WNT3 | WNT4 | WNT5B | WNT6 | WNT7B | WNT8B |
| WRN | WSB1 | WT1 | WWOX | WWTR1 | XBP1 | XIAP | |
| XKR3 | XPA | XPC | XPO1 | XRCC6 | YAP1 | YPEL5 | YTHDF2 |
| YWHAE | YY1AP1 | ZAP70 | ZBTB16 | ZC3H7A | ZC3H7B | ZFP64 | |
| ZFPM2 | ZFYVE19 | ZIC2 | ZMIZ1 | ZMYM2 | ZMYM3 | ZMYND11 | ZNF207 |
| ZNF217 | ZNF24 | ZNF331 | ZNF384 | ZNF444 | ZNF521 | ZNF585B | |
| ZNF687 | ZNF703 | ZRSR2 | | | | | |

After the sequence run finished, data were run through bcl2fastq2 Software v.2.20.0 to de-multiplex. Subsequent sequence analyses included Dragen 3.8 RNA seq pipeline for fusion calls, Salmon v1.4.0 for determination of expression levels (measured in TPM), cnvkit for determination of CNV calls, and RNA-Seq Alignment v.2.0.2 - BaseSpace Sequence Hub App for VCF to get mutation calls.

Patient samples: Peripheral blood samples of 160 individuals were collected in EDTA tubes. Of these individuals, 31 were healthy control and 129 were patients with a history of myeloid (22), lymphoid (73), or solid tumors (34) as shown in **Table** 2 below. Total nucleic acid was extracted from 1 ml of plasma of these samples, reverse transcription and target enrichment using the genes of Table 1 was performed as described above.

**Table 2**

| Normal | Lymphoid | Myeloid | Solid tumors | Total |
|---|---|---|---|---|
| 31 | 73 | 22 | 34 | 160 |

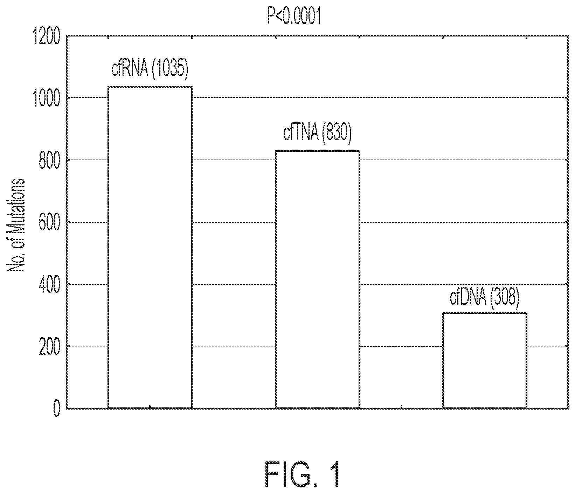

Sequence analysis of each patient's target enriched cDNA libraries (based on cfTNA and cfRNA fraction for each patient) revealed that significantly higher numbers of mutations can be detected form cfRNA fractions. As can be clearly seen from **FIG.1****,** significantly more mutations were detected using cfRNA only as compared with cfTNA using the same gene enrichment panel. Notably, the number of mutations detected in a routine testing based on a known DNA panel with 275 genes, identified substantially less mutants. It is noteworthy that the number of mutations detected in cfRNA testing was significantly higher than that when cfTNA or cfDNA was used. The number of genes used in testing cfRNA and cfTNA was also significantly higher (1485 genes) than that used in the DNA (275 genes). However, since the 275 gene panel included most of the clinically relevant oncogenic genes, only 45 mutations were detected in RNA testing in genes that were not included in the 275 genes. In fact, these 45 mutations were concentrated in 27 genes. In view of these finding, it can be clearly seen that cfRNA analysis is more sensitive and informative. However, cfRNA is at a disadvantage for detection of low-expression or unexpressed mutations or where RNA is rapidly degraded beyond isolation limits as is shown in more detail below.

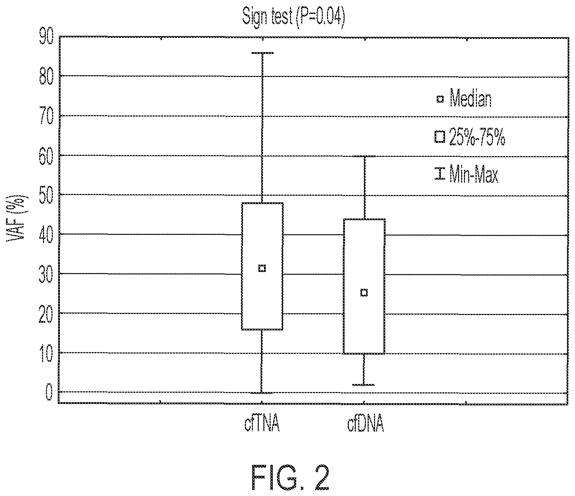

In a further set of analysis, the inventor investigated the influence of cfRNA and cfTNA on variant allele frequency (VAF)/sensitivity. More specifically, the inventor compared the VAF between cfTNA and cfDNA when mutations were detected in both methods. As can be seen in **FIG.2****,** there is a significant difference between the two methods in the level of VAF (sign test [null hypothesis test] P=0.04). This comparison clearly demonstrates substantially higher sensitivity in detected mutations when cfTNA is used. While not limiting to a specific theory or hypothesis, the inventor contemplates that such difference may be attributable to the cfRNA fraction in the cfTNA.

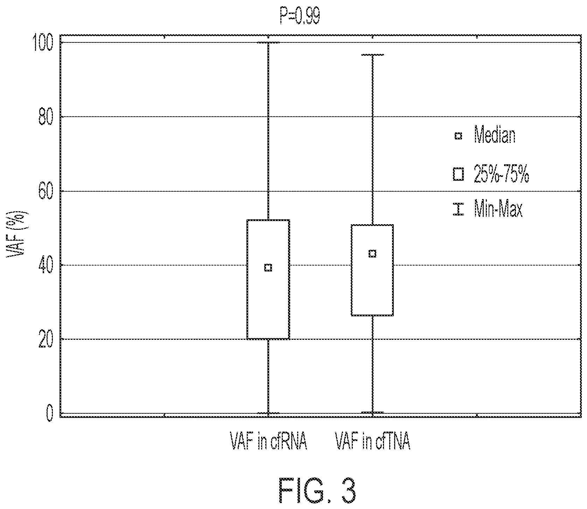

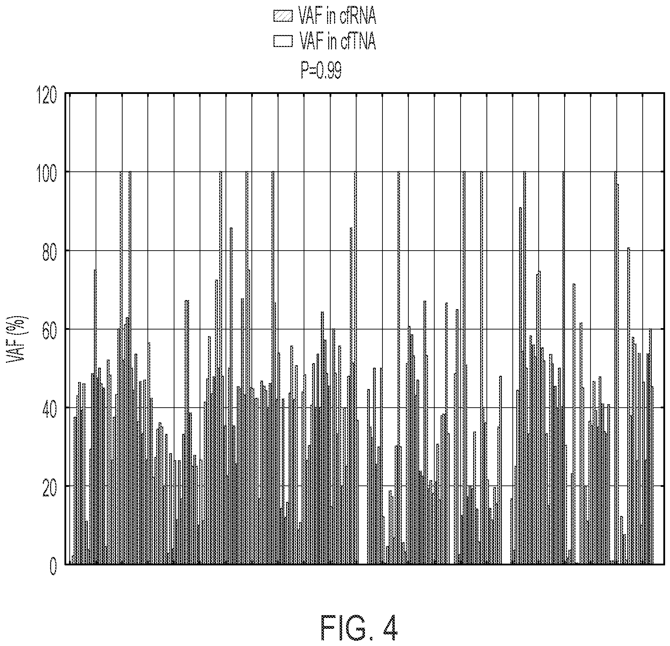

The inventor then set out to determine potential benefits for comprehensive detection of mutations when both cfTNA and cfRNA were used. As already shown above, a higher number of mutations were detected when cfRNA was used as compared to cfTNA or cfDNA. However, the inventor discovered that certain mutations could be detected in cfTNA, but not in cfRNA. Such difference is most likely due to the phenomenon that early termination of translation due to mutations may lead to increased degradation of the mutant RNA. In addition to such observation, (improper) splicing mutations may also lead to early degradation of RNA. Overall there was no difference in VAF between cfRNA and cfTNA when the mutations are detected in both analysis as can be seen from **FIG.3****.** However, some mutations were clearly detected at higher levels in cfRNA as compared with cfTNA and *vice versa* as is evident from **FIG.4****.** The examples below demonstrate that there are significant numbers of mutations that are detected in cfDNA but not in cfRNA. **Table 3** shows example of mutation detected in cfTNA, but not in cfRNA. Note the high proportion of mutations leading to termination. The remaining mutations likely highly destabilizing.

| **Gene** | **HGVSc** | **HGVSp** | **VAF in cfRNA** | **VAF in cfTNA** | **Amino Acid change** |
|---|---|---|---|---|---|
| TET2 | NM_001127208.2:c.2737C>T | NP_001120680.1:p.Gln913Ter | 0 | 0.995 | Q/* |
| PDGFRB | NM_002609.3:c.1403A>C | NP_002600.1:p.Asn468Thr | 0 | 1.19 | N/T |
| TRAF3 | NM_003300.3:c.1688C>T | NP_003291.2:p.Ser563Leu | 0 | 1.87 | F/S |
| DNMT3A | NM_175629.2:c.2338A>T | NP_783328.1:p.Ile780Phe | 0 | 0.33 | I/F |
| KMT2C | NM_170606.2:c.4046G>A | NP_733751.2:p.Arg1349Gln | 0 | 55 | R/Q |
| DNMT3A | NM_175629.2:c.1792C>T | NP_783328.1:p.Arg598Ter | 0 | 2.25 | R/* |
| CHEK2 | NM_001005735.1:c.668G>A | NP_001005735.1:p.Arg223His | 0 | 50 | R/H |
| MYD88 | NM_001172567.1:c.16_34delGCTGAGGCTCCAGGACCGC | NP_001166038.1:p.Ala6ProfsTer39 | 0 | 51.06 | DRAEAPG/X |
| BRIP1 | NM_032043.2:c.1871C>T | NP_114432.2:p.Ser624Leu | 0 | 16.67 | S/L |
| PPM1D | NM_003620.3:c.1538T>A | NP_003611.1:p.Leu513Ter | 0 | 2.04 | L/* |
| LRP1B | NM_018557.2:c.513C>G | NP_061027.2:p.Asn171Lys | 0 | 20 | N/K |
| PDGFRB | NM_002609.3:c.1000C>T | NP_02600.1:p.Arg334Trp | 0 | 51.67 | R/W |
| NOTCH2 | NM_024408.3:c.6424T>C | NP_077719.2:p.Ser2142Pro | 0 | 45.83 | S/P |
| BCR | NM_004327.3:c.3286A>G | NP_004318.3:p.Thr1096Ala | 0 | 12.2 | T/A |
| NF1 | NM_001042492.2:c.8128G>T | NP_001035957.1:p.Gly2710Cys | 0 | 57.14 | G/C |
| EZH2 | NM_004456.4:c.1936T>C | NP_004447.2:p.Tyr646His | 0 | 9.52 | Y/H |
| PTEN | NM_000314.4:c.492+2T>G | | 0 | 53.85 | |
| CD79B | NM_001039933.1:c.589T>A | NP_001035022.1:p.Tyr197Asn | 0 | 10.14 | Y/N |
| STAG2 | NM_001042749.1:c.1840C>T | NP_001036214.1:p.Arg614Ter | 0 | 36.56 | R/* |
| TET2 | NM_001127208.2:c.2839C>T | NP_001120680.1:p.Gln947Ter | 0 | 7.82 | Q/* |
| ASXL1 | NM_015338.5:c.2564_2567delATTG | NP_056153.2:p.Asp855AlafsTer11 | 0 | 22.14 | TD/X |
| FANCA | NM_000135.2:c.2T>C | NP_000126.2:p.Met1? | 0 | 26.09 | M/T |
| ROS1 | NM_002944.2:c.3000A>T | NP_002935.2:p.Leu1000Phe | 0 | 64.86 | L/F |
| CHEK2 | NM_001005735.1:c.1229delC | NP_001005735.1:p.Thr410MetfsTer15 | 0 | 47.92 | T/X |
| FANCC | NM_000136.2:c.456+4A>T | | 0 | 68.57 | |
| FANCC | NM_000136.2:c.456+4A>T | | 0 | 66.67 | |
| CHEK2 | NM_001005735.1:c.1229delC | NP_001005735.1:p.Thr410MetfsTer15 | 0 | 36.06 | T/X |
| DNMT3A | NM_175629.2:c.2479-2A>G | | 0 | 21.35 | |
| SRSF2 | NM_003016.4:c.284C>A | NP_003007.2:p.Pro95His | 0 | 37.5 | P/H |
| ASXL1 | NM_015338.5:c.3041delG | NP_056153.2:p.Ser1014MetfsTer10 | 0 | 45.51 | |
| TET2 | NM_001127208.2:c.4628delG | NP_001120680.1:p.Arg1543AsnfsTer28 | 0 | 46.25 | |
| NRAS | NM_002524.4:c.38G>T | NP_002515.1:p.Gly13Val | 0 | 17.86 | |
| SF3B1 | NM_012433.2:c.1549C>T | NP_036565.2:p.Arg517Cys | 0 | 7.69 | |
| FLT4 (germline) | NM_182925.4:c.2563G>A | NP_891555.2:p.Ala855Thr | 0 | 48.27 | A/T |
| PIK3CA | NM_006218.2:c.3140A>G | NP_006209.2:p.His1047Arg | 0 | 12.2 | H/R |
| ESR1 | NM_001122742.1:c.1610A>C | NP_001116214.1:p.Tyr537Ser | 0 | 12.02 | Y/S |
| TP53 | NM_000546.5:c.811G>T | NP_000537.3:p.Glu271Ter | 0 | 22.66 | E/* |
| FLT3-ITD | NM_004119.2:c.1790_1807dupATGAATATGATCTCAAAT | NP_004110.2:p.Tyr597_Lys602dup | 0 | 24.05 | W/YEYDLKW |
| NPM1 | NM_002520.6:c.863_864insCATG | NP_002511.1:p.Trp288CysfsTer12 | 0 | 100 | -/CX |
| BAP1 | NM_004656.3:c.206delC | NP_004647.1:p.Thr69SerfsTer3 | 0 | 25.65 | T/X |
| CREBBP | NM_004380.2:c.5218dupC | NP_004371.2:p.His1740ProfsTer2 | 0 | 16.67 | H/PX |
| KEAP1 | NM_203500.1:c.811G>T | NP_987096.1:p.Val271Leu | 0 | 19.46 | V/L |
| CD79B | NM_001039933.1:c.498G>T | NP_001035022.1:p.Gln166His | 0 | 8.79 | Q/H |
| SETBP1 | NM_015559.2:c.4691delC | NP_056374.2:p.Pro1564HisfsTer16 | 0 | 75 | P/X |
| DNMT3A | NM_175629.2:c.2130C>A | NP_783328.1:p.Cys710Ter | 0 | 12.88 | C/* |
| STAG2 | NM_001042749.1:c.3395T>A | NP_001036214.1:p.Leu1132Ter | 0 | 0.21 | L/* |
| ARID1B | NM_020732.3:c.679G>C | NP_065783.3:p.Val227Leu | 0 | 18.22 | V/L |
| ARID1B | NM_020732.3:c.680T>C | NP_065783.3:p.Val227Ala | 0 | 17.23 | V/A |
| SMC3 | NM_005445.3:c.2182T>G | NP_005436.1:p.Phe728Val | 0 | 1.09 | F/V |
| IDH2 | NM_002168.2:c.419G>A | NP_002159.2:p.Arg140Gln | 0 | 0.5 | R/Q |
| ASXL1 | NM_015338.5:c.1934dupG | NP_056153.2:p.Gly646TrpfsTer12 | 0 | 3.64 | -/X |
| NOTCH2 | NM_024408.3:c.7163C>G | NP_077719.2:p.Ser2388Ter | 0 | 2.47 | S/* |
| CREBBP | NM_004380.2:c.379_382dupGATT | NP_004371.2:p.Ser128Ter | 0 | 1.82 | |
| SRSF2 | NM_003016.4:c.284C>T | NP_003007.2:p.Pro95Leu | 0 | 5 | P/L |

In addition to significantly improved detection of mutants and VAF determination, the inventor also demonstrated that systems and methods presented herein are suitable for the accurate prediction of immunophenotype, immunohistochemistry profile, and diagnosis and measurement of biomarkers via quantitative analysis of cfRNA expression. More specifically, the inventor discovered that targeted RNA sequencing from the cfRNA and/or cfTNA fractions allows measuring expression levels of proteins that are typically used for immunophenotyping and immunohistochemistry (IHC) profiling, and to use the expression levels of selected proteins as biomarkers in the diagnosis, prediction of prognosis, and monitoring of various diseases and cancer as RNA levels typically reflect protein levels and so may be useful as surrogate for measurement of actual protein expression.

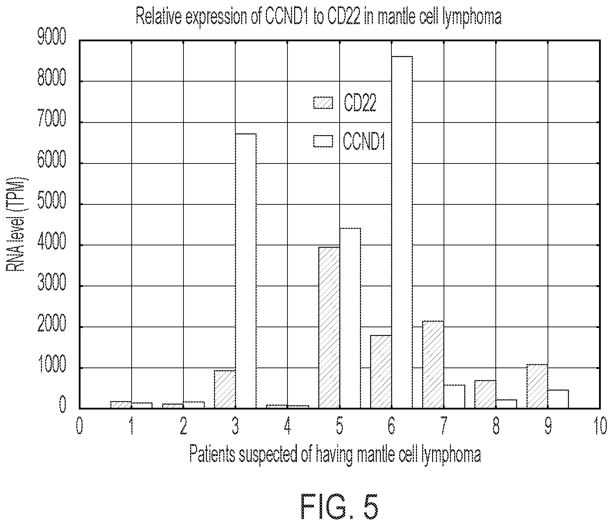

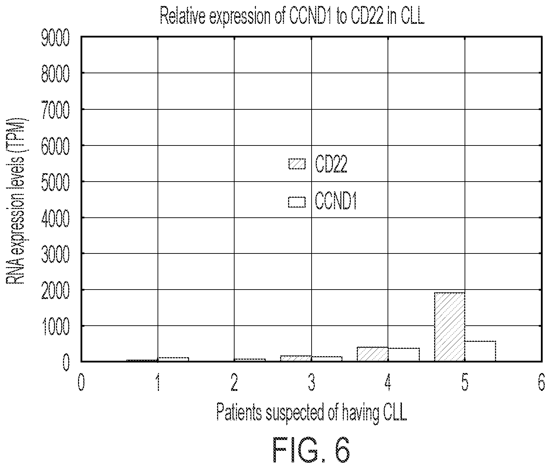

For example, the expression level of CCND1 (especially relative to CD22) can be used as a diagnostic marker for mantle cell lymphoma. Using samples of the tested patient population, **FIG.5** demonstrates that the expression level (and especially relative expression level vis-à-vis general B-cell marker CD22) can accurately diagnose presence of mantle cell lymphoma for individuals #3 and #6. In contrast none of the chronic lymphocytic leukemia (CLL) samples showed similar high CCND1:CD22 ratios as can be readily taken from FIG.6. Thus, it should be appreciated that expression level data from cfRNA analyses can accurately differentiate distinct lymphatic cancer types.

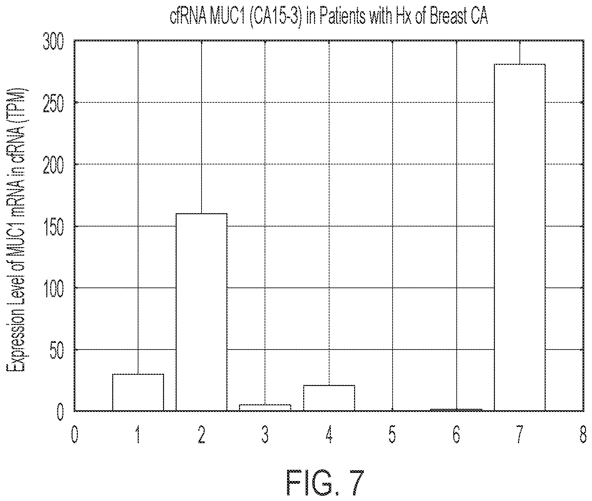

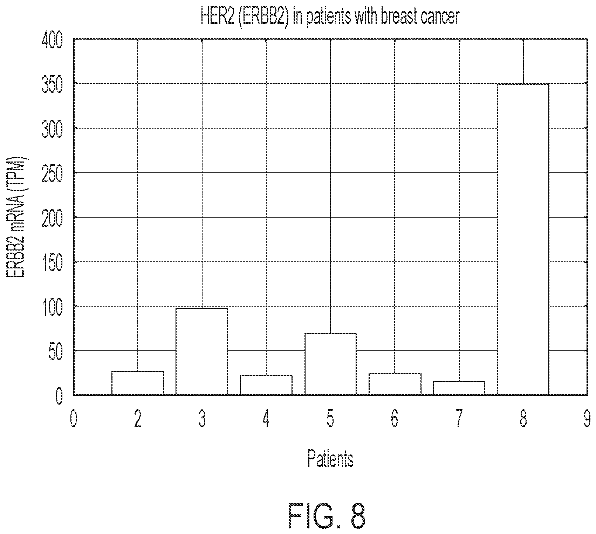

Similarly for solid tumors, expression levels of CA15-3 (MUC1) in cfRNA samples can be used to distinguish samples with active breast cancer from other conditions as can be seen from patient #2 and #7 of FIG.7. Also these patients with breast cancer and high ERBB2 (HER2) could be distinguished by evaluating ERBB2 mRNA in peripheral blood cfRNA as is clearly shown in FIG.8.

In still further series of experiments, the inventor used cfRNA expression profiling with machine learning for the diagnosis of various types of cancers and for early detection. In one example, the inventor used cfRNA expression levels as determined by TPM (Transcripts Per Kilobase Million) profiling with a machine learning algorithm for predicting the presence or absence of cancer. In such system, the expression levels of the NGS targeted genes were analyzed using a machine learning system developed to predict the presence of a specific cancer as well as to determine the genes needed for this prediction. A subset of genes relevant to cancer was automatically selected for the classification system, based on a k-fold cross validation procedure (with k=10). For an individual gene, a naive Bayesian classifier was constructed on the training of k-1 subsets and tested on the other testing subset. The training and testing subsets were then rotated, and the average of the classification errors was used to measure the relevancy of the gene. The classification system was trained with the selected subset of most relevant genes, and Geometric Mean Naive Bayesian (GMNB) was employed as the classifier to predict a specific cancer. GMNB is a generalized naive Bayesian classifier by applying a geometric mean to the likelihood product, which eliminates the underflow problem commonly associated with the standard Naive Bayesian classifiers with high dimensionality. The processes of gene selection and cancer classification were applied iteratively to obtain an optimal classification system and a subset of genes relevant to the specific cancer of interest.

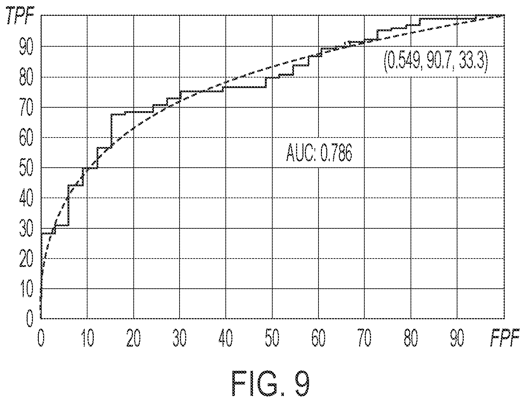

Predicting the presence of any cancer: Using the measured expression levels with the machine learning approached described above, analysis of the 160 patients described above showed that one can indeed distinguish patients with cancer with an area under the curve (AUC) of 0.786 using the 1450 genes of Table 1 as is shown in FIG.9. This prediction is expected to improve by adding mutation profiling to this system.

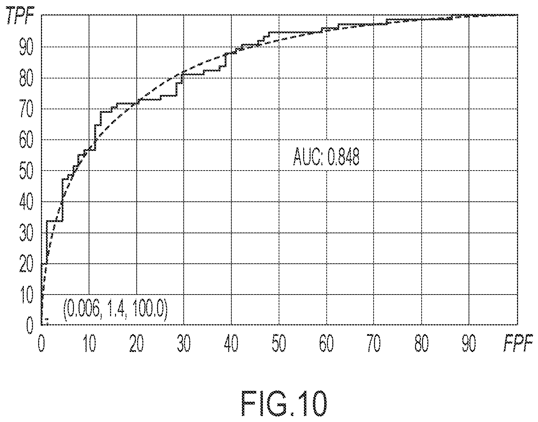

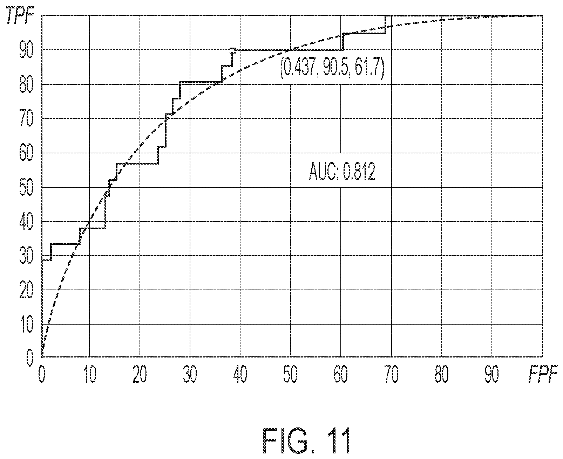

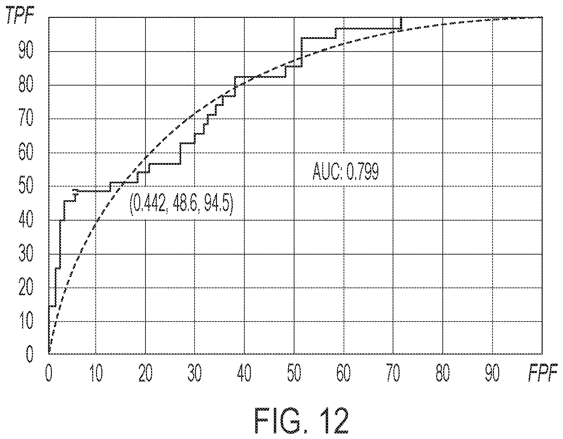

Predicting the presence of a specific cancer: The cfRNA expression profiling along with developed machine learning model can also predict the specific cancer. For example, the inventor distinguished patients with lymphoid neoplasms (diffuse large B-cell lymphoma, mantle cell lymphoma, chronic lymphocytic leukemia, acute lymphoblastic leukemia) with an AUC of 0.848 using 650 genes as shown in **FIG.10****.** Similarly, the inventor distinguished patients with myeloid cancer (acute myeloid leukemia, myelodysplastic syndrome, myeloproliferative neoplasms, etc.) with an AUC of 0.812 using 1450 genes as shown in **FIG.11****.** Likewise, the inventor distinguished patients with solid tumors (breast, lung, ovary, etc.) with AUC of 0.799 using 950 genes as shown in **FIG.12****.**

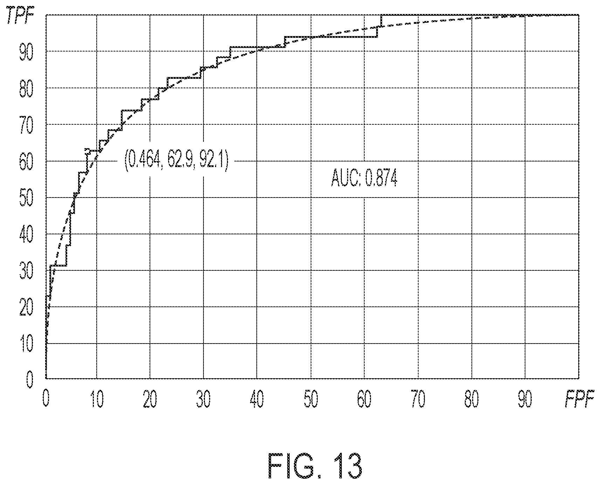

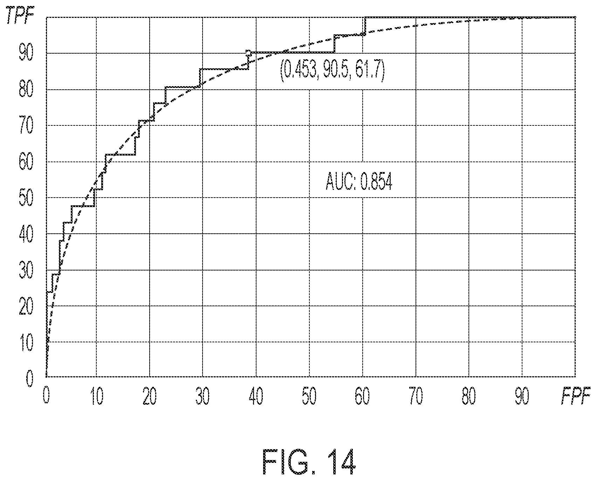

As will be readily appreciated, all of these analyses can be improved if a mutation profile is added to the cfRNA expression profile. Furthermore, prediction can also be improved by adding the levels of cfTNA as measured by TPM, which will encompass any genomic CNV (copy number variation), to the variables used for prediction of the presence of a specific cancer. For example, solid tumors prediction AUC improved significantly from 0.799 to 0.874 when the cfTNA was added to the algorithm as can be seen from **FIG.13****.** In the same way, myeloid cancer prediction improved significantly by adding the cfTNA data as is evident from the improved AUC (from 0.812 to 0.854) as shown in **FIG.14****.** Thus, it should once more be recognized that the use of cfRNA and cfDNA will significantly improve clinical analysis, which in turn will improve treatment and prevention in an individual.

In yet further examples, the inventor also used cfRNA and cfTNA in the detection of cytogenetic changes. Typically, cytogenetic abnormalities are chromosomal translocations or structural gains and/or losses. Using contemplated systems and methods, analysis of both, cfRNA and cfTNA, enables complete cytogenetic analysis.

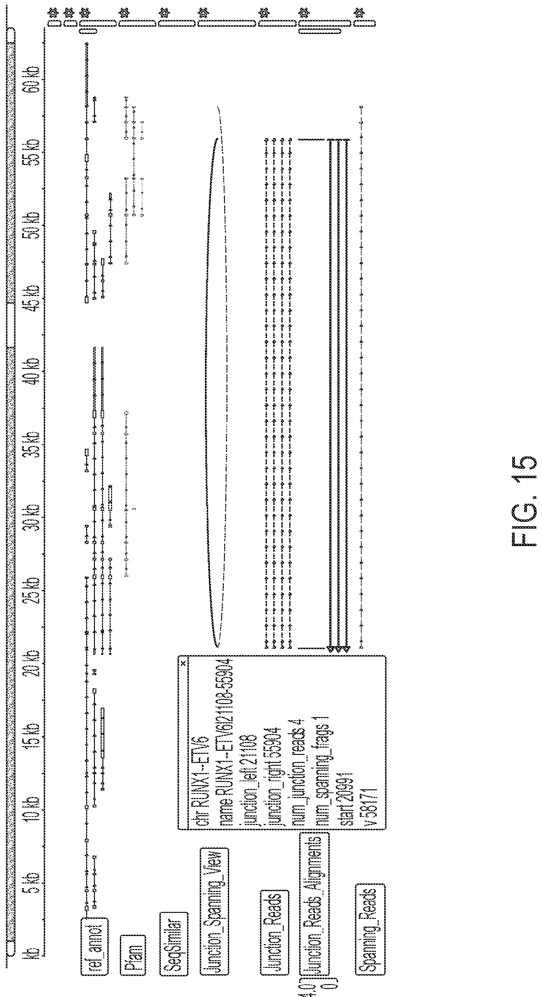

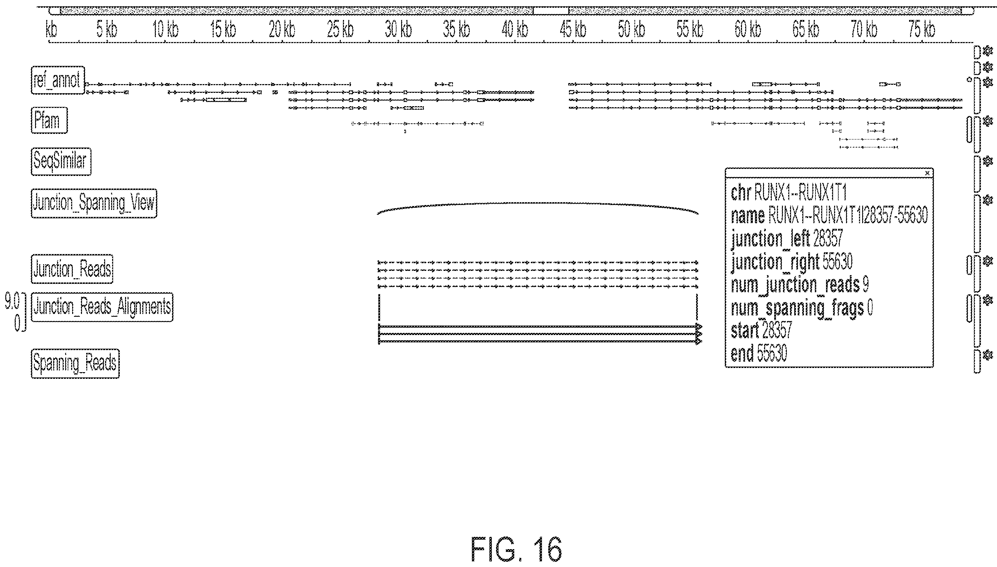

For example, chromosomal translocations can be detected from RNA fusion resulting from chromosomal translocations, and the inventor discovered that RNA fusion products were significantly more reliable in detecting these chromosomal translocations. Furthermore, when RNA sequencing is used, translocations can be detected irrespective of the partner gene. By cfRNA sequencing the inventor was able to detect various fusion mRNA. For example, the inventor was able to detect t(12;21)(p13;q22)RUNX1-ETV6 in a pediatric patient with acute lymphoblastic leukemia as can be seen in **FIG.15****.** In another example, t(8;21)(q22;q22) RUNX1-RUNX1T1 was detected in a patient with acute myeloid leukemia as can be taken from **FIG.16****.**

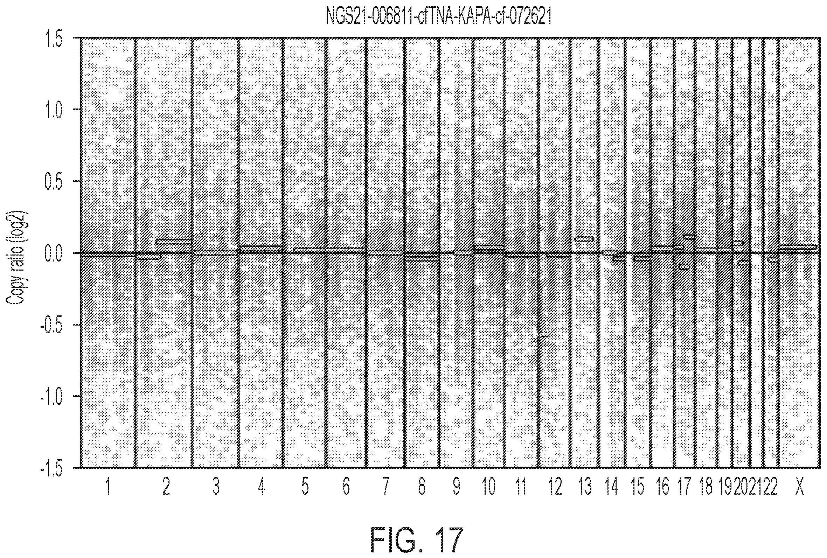

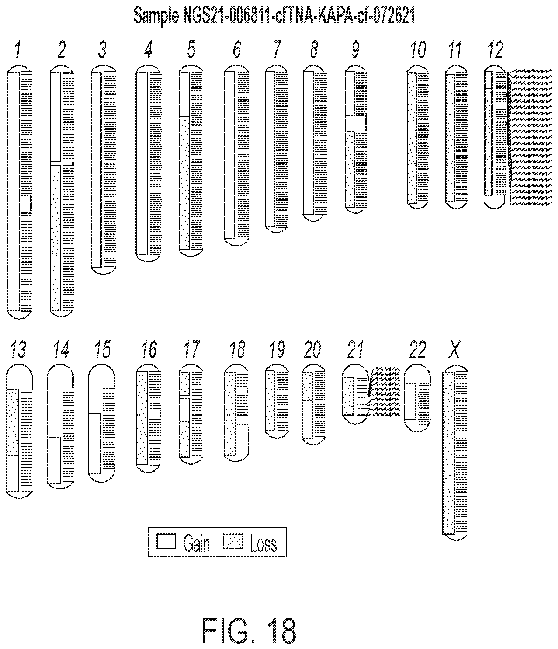

Moreover, contemplated systems and methods will also enable the detection of various chromosomal structural abnormalities. For example, using cfTNA sequencing allows analysis of chromosomal structural abnormalities using standard approaches like CNVkit approach. **FIG.17** and **FIG.18** show cfTNA data in a pediatric patient with acute lymphoblastic leukemia, confirming that cfRNA and cfTNA analysis can perform complete cytogenetic analysis for chromosomal translocations and/or structural gains or loses.

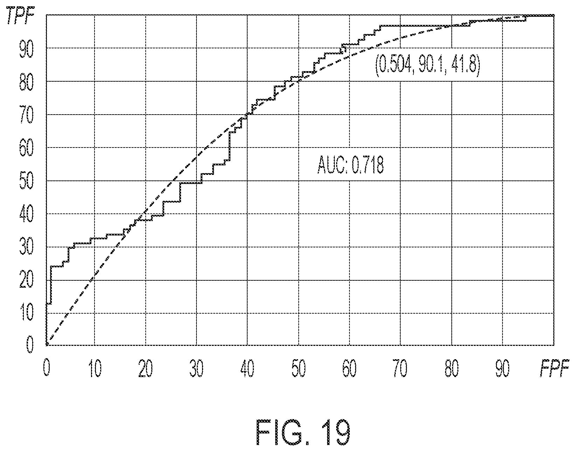

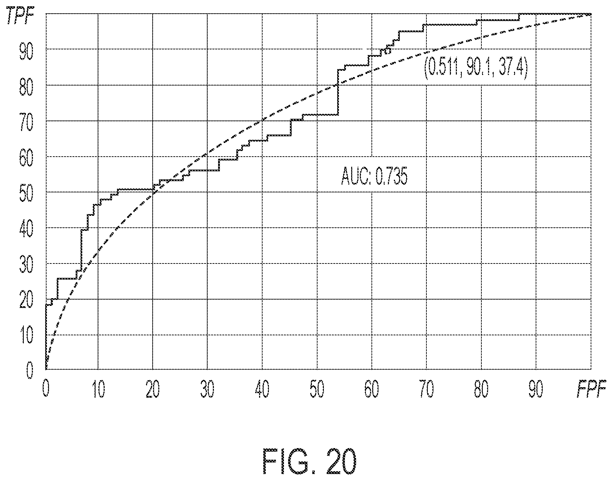

Finally, the inventor also discovered that using expression profiles of cfRNA and/or cfTNA can be employed for the detection of minimal residual disease. More specifically, using expression profile of cfRNA or cfTNA along with a machine learning approach, enabled prediction of patients with active cancer that shows mutations in peripheral blood circulation. Using cfRNA, the inventor was able to predict the presence of mutations in circulation with AUC of 0.718 as shown in **FIG.19****,** while using cfTNA, the inventor was able to predict the presence of mutations in circulation with AUC of 0.735 as is shown in **FIG.20****.**

In view of the above, it should therefore be appreciated that quantifying both RNA and DNA (and especially cfTNA/cfRNA) in a sample and using both for developing biomarkers for the prediction of biological events (diagnosis, response to therapy, prognosis...) provides a novel and highly sensitive too for molecular medicine. Indeed, one significant advantage of quantifying DNA in the same fashion as with RNA is to evaluate genomic gains and losses. When this is added to RNA information, the discovery of new biomarkers is improved significantly. Moreover, it should be appreciated that the systems and methods presented herein keep the RNA and use hybrid capture to pull out cDNA/RNA and exons from the DNA in the sample.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description and attached claims are approximations that can vary depending upon the desired properties sought to be obtained by a particular embodiment. The recitation of ranges of values herein is merely intended to serve as a shorthand method of referring individually to each separate value falling within the range.

As used herein, the term "administering" a pharmaceutical composition or drug refers to both direct and indirect administration of the pharmaceutical composition or drug, wherein direct administration of the pharmaceutical composition or drug is typically performed by a health care professional (e.g., physician, nurse, etc.), and wherein indirect administration includes a step of providing or making available the pharmaceutical composition or drug to the health care professional for direct administration (e.g., via injection, infusion, oral delivery, topical delivery, etc.). It should further be noted that the terms "prognosing" or "predicting" a condition, a susceptibility for development of a disease, or a response to an intended treatment is meant to cover the act of predicting or the prediction (but not treatment or diagnosis of) the condition, susceptibility and/or response, including the rate of progression, improvement, and/or duration of the condition in a subject.

All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

It should be noted that any language directed to a computer should be read to include any suitable combination of computing devices, including servers, interfaces, systems, databases, agents, peers, engines, modules, controllers, or other types of computing devices operating individually or collectively. One should appreciate the computing devices comprise a processor configured to execute software instructions stored on a tangible, non-transitory computer readable storage medium (e.g., hard drive, solid state drive, RAM, flash, ROM, etc.). The software instructions preferably configure the computing device to provide the roles, responsibilities, or other functionality as discussed below with respect to the disclosed apparatus. In especially preferred embodiments, the various servers, systems, databases, or interfaces exchange data using standardized protocols or algorithms, possibly based on HTTP, HTTPS, AES, public-private key exchanges, web service APIs, or other electronic information exchanging methods. Data exchanges preferably are conducted over a packet-switched network, the Internet, LAN, WAN, VPN, or other type of packet switched network.

As used in the description herein and throughout the claims that follow, the meaning of "a," "an," and "the" includes plural reference unless the context clearly dictates otherwise. Also, as used in the description herein, the meaning of "in" includes "in" and "on" unless the context clearly dictates otherwise. As also used herein, and unless the context dictates otherwise, the term "coupled to" is intended to include both direct coupling (in which two elements that are coupled to each other contact each other) and indirect coupling (in which at least one additional element is located between the two elements). Therefore, the terms "coupled to" and "coupled with" are used synonymously.

In interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of something selected from the group consisting of A, B, C .... and N, the text should be interpreted as requiring only one element from the group, not A plus N, or B plus N, etc.

## Claims

1. A method of manipulating nucleic acids from a cell-free fluid, comprising:
obtaining cell-free total nucleic acid (cfTNA) from a biological fluid;
subjecting a first portion of the cfTNA to DNAse digestion to so generate a cfRNA fraction of the cfTNA;
subjecting both, the cfRNA fraction of the cfTNA and a second portion of the cfTNA, to reverse transcription, adapter ligation, and amplification to thereby generate respective first and second cDNA libraries;
subjecting each of the first and second cDNA libraries to target enrichment that enriches a plurality of target cDNAs to thereby generate respective first and second target-enriched cDNA libraries.

2. The method of claim 1, wherein the cfTNA comprises cfRNA fragments having a size of between 30 and 250 bases, and cfDNA fragments having a size of between 75 and 400 bases.

3. The method of claim 1, wherein the cfTNA comprises cfRNA fragments having a size of between 17 and 200 bases, and cfDNA fragments having a size of between 50 and 300 bases.

4. The method of any one of claim 2 or claim 3, wherein the cfRNA fragments and the cfDNA fragments constitute together at least 30% of all cfTNA.

5. The method of any one of claim 2 or claim 3, wherein the cfRNA fragments and the cfDNA fragments constitute together at least 40% of all cfTNA.

6. The method of any one of the preceding claims, wherein the step of obtaining the cfTNA from the biological fluid comprises simultaneous isolation of cfRNA and cfDNA.

7. The method of any one of the preceding claims, wherein the reverse transcription comprises a step of random priming for the first strand synthesis.

8. The method of any one of the preceding claims, wherein the target enrichment uses for each target cDNA a plurality of hybridization probes that bind to the target cDNA at respective different positions.

9. The method of claim 8, wherein the plurality of hybridization probes bind to the target cDNA in a tiled fashion, and wherein the plurality of hybridization probes provide a tiling density of at least 2x.

10. The method of claim 8, wherein the plurality of hybridization probes bind to the target cDNA in a tiled fashion with a step length of n, wherein n is an integer between 1-10.

11. The method of any one of the preceding claims, further comprising a step of amplifying the first and the second target-enriched cDNA libraries.

12. The method of any one of the preceding claims, further comprising a step of sequencing the first and the second target-enriched cDNA libraries or sequencing the amplified first and the second target-enriched cDNA libraries.

## Patentansprüche

1. Verfahren zum Manipulieren von Nukleinsäuren aus einem zellfreien Fluid, umfassend:
Erhalten von zellfreier Gesamtnukleinsäure (cfTNA) aus einem biologischen Fluid;
Aussetzen eines ersten Teils der cfTNA gegenüber DNAse-Verdauung, um so eine cfRNA-Fraktion der cfTNA zu erzeugen;
Aussetzen sowohl der cfRNA-Fraktion der cfTNA als auch eines zweiten Teils der cfTNA gegenüber reverser Transkription, Adapterligation und Amplifikation, um dadurch eine jeweilige erste und zweite cDNA-Bibliothek zu erzeugen;
Aussetzen von jeder von der ersten und der zweiten cDNA-Bibliothek gegenüber Zielanreicherung, die eine Vielzahl von Ziel-cDNAs anreichert, um dadurch eine jeweilige erste und zweite zielangereicherte cDNA-Bibliothek zu erzeugen.

2. Verfahren nach Anspruch 1, wobei die cfTNA cfRNA-Fragmente mit einer Größe zwischen 30 und 250 Basen und cfDNA-Fragmente mit einer Größe zwischen 75 und 400 Basen umfasst.

3. Verfahren nach Anspruch 1, wobei die cfTNA cfRNA-Fragmente mit einer Größe zwischen 17 und 200 Basen und cfDNA-Fragmente mit einer Größe zwischen 50 und 300 Basen umfasst.

4. Verfahren nach einem von Anspruch 2 oder Anspruch 3, wobei die cfRNA-Fragmente und die cfDNA-Fragmente zusammen zumindest 30 % aller cfTNA ausmachen.

5. Verfahren nach einem von Anspruch 2 oder Anspruch 3, wobei die cfRNA-Fragmente und die cfDNA-Fragmente zusammen zumindest 40 % aller cfTNA ausmachen.

6. Verfahren nach einem der vorhergehenden Ansprüche, wobei der Schritt des Erhaltens der cfTNA aus dem biologischen Fluid gleichzeitige Isolierung von cfRNA und cfDNA umfasst.

7. Verfahren nach einem der vorhergehenden Ansprüche, wobei die reverse Transkription einen Schritt des Zufallsprimings für die Synthese des ersten Strangs umfasst.

8. Verfahren nach einem der vorhergehenden Ansprüche, wobei die Zielanreicherung für jede Ziel-cDNA eine Vielzahl von Hybridisierungssonden verwendet, die an die Ziel-cDNA an jeweiligen unterschiedlichen Positionen bindet.

9. Verfahren nach Anspruch 8, wobei die Vielzahl von Hybridisierungssonden an die Ziel-cDNA auf eine gekachelte Weise bindet und wobei die Vielzahl von Hybridisierungssonden eine Kacheldichte von zumindest 2x bereitstellt.

10. Verfahren nach Anspruch 8, wobei die Vielzahl von Hybridisierungssonden an die Ziel-cDNA auf eine gekachelte Weise mit einer Schrittlänge von n bindet, wobei n eine ganze Zahl zwischen 1-10 ist.

11. Verfahren nach einem der vorhergehenden Ansprüche, ferner umfassend einen Schritt des Amplifizierens der ersten und der zweiten zielangereicherten cDNA-Bibliothek.

12. Verfahren nach einem der vorhergehenden Ansprüche, ferner umfassend einen Schritt des Sequenzierens der ersten und der zweiten zielangereicherten cDNA-Bibliothek oder des Sequenzierens der amplifizierten ersten und zweiten zielangereicherten cDNA-Bibliothek.

## Revendications

1. Procédé de manipulation d'acides nucléiques provenant d'un fluide acellulaire, comprenant :
l'obtention d'acide nucléique total acellulaire (cfTNA) à partir d'un fluide biologique ;
la soumission d'une première partie du cfTNA à une digestion par DNAse pour générer ainsi une fraction de cfRNA du cfTNA ;
la soumission, à la fois, de la fraction de cfRNA du cfTNA et d'une seconde partie du cfTNA, à une transcription inverse, à une ligature d'adaptateurs et à une amplification pour générer ainsi des première et seconde banques d'ADNc respectives ;
la soumission de chacune des première et seconde banques d'ADNc à un enrichissement de cibles qui enrichit une pluralité d'ADNc cibles pour générer ainsi des première et seconde banques d'ADNc enrichies en cibles respectives.

2. Procédé de la revendication 1, dans lequel le cfTNA comprend des fragments de cfRNA ayant une taille comprise entre 30 et 250 bases, et des fragments de cfDNA ayant une taille comprise entre 75 et 400 bases.

3. Procédé de la revendication 1, dans lequel le cfTNA comprend des fragments de cfRNA ayant une taille comprise entre 17 et 200 bases, et des fragments de cfDNA ayant une taille comprise entre 50 et 300 bases.

4. Procédé de l'une quelconque de la revendication 2 ou la revendication 3, dans lequel les fragments de cfRNA et les fragments de cfDNA constituent ensemble au moins 30 % de la totalité de cfTNA.

5. Procédé de l'une quelconque de la revendication 2 ou la revendication 3, dans lequel les fragments de cfRNA et les fragments de cfDNA constituent ensemble au moins 40 % de la totalité de cfTNA.

6. Procédé de l'une quelconque des revendications précédentes, dans lequel l'étape d'obtention du cfTNA à partir du fluide biologique comprend l'isolement simultané de cfRNA et de cfDNA.

7. Procédé de l'une quelconque des revendications précédentes, dans lequel la transcription inverse comprend une étape d'amorçage aléatoire pour la synthèse de premier brin.

8. Procédé de l'une quelconque des revendications précédentes, dans lequel l'enrichissement de cibles utilise, pour chaque ADNc cible, une pluralité de sondes d'hybridation qui se lient à l'ADNc cible à des positions différentes respectives.

9. Procédé de la revendication 8, dans lequel la pluralité de sondes d'hybridation se lie à l'ADNc cible selon une disposition en tuilage, et dans lequel la pluralité de sondes d'hybridation fournit une densité de tuilage d'au moins 2x.

10. Procédé de la revendication 8, dans lequel la pluralité de sondes d'hybridation se lie à l'ADNc cible selon une disposition en tuilage avec une longueur de pas de n, dans lequel n est un nombre entier compris entre 1 et 10.

11. Procédé de l'une quelconque des revendications précédentes, comprenant en outre une étape d'amplification des première et seconde banques d'ADNc enrichies en cibles.

12. Procédé de l'une quelconque des revendications précédentes, comprenant en outre une étape de séquençage des première et seconde banques d'ADNc enrichies en cibles ou de séquençage des première et seconde banques d'ADNc enrichies en cibles amplifiées.
